(19) Europäisches Patentamt
European Patent Office
Office européen des brevets

(11) Veröffentlichungsnummer : **0 009 201**
**B1**

(12) # EUROPÄISCHE PATENTSCHRIFT

(45) Veröffentlichungstag der Patentschrift : **02.12.81**

(51) Int. Cl.³ : **C 07 H 15/22**// A61K31/70, C07H23/00

(21) Anmeldenummer : **79103385.5**

(22) Anmeldetag : **11.09.79**

(54) **Selektiv geschützte 4,6-Di-O-(Aminoglykosyl)-1,3-diamino-cyclitole, Verfahren zu deren Herstellung und deren Verwendung als Zwischenprodukte.**

(30) Priorität : **20.09.78 DE 2840907**

(43) Veröffentlichungstag der Anmeldung : **02.04.80 (Patentblatt 80/07)**

(45) Bekanntmachung des Hinweises auf die Patenterteilung : **02.12.81 Patentblatt 81/48**

(84) Benannte Vertragsstaaten : **AT BE CH DE FR GB IT NL SE**

(56) Entgegenhaltungen :
**DE - A - 2 631 462**
**EP - A - 0 000 057**
**EP - A - 0 000 800**
**EP - A - 0 002 450**
**FR - A - 2 240 015**

(73) Patentinhaber : **BAYER AG**
**Zentralbereich Patente, Marken und Lizenzen**
**D-5090 Leverkusen 1, Bayerwerk (DE)**

(72) Erfinder : **Voss, Eckart, Dr.**
**Morgengraben 10**
**D-5000 Köln 80 (DE)**
Erfinder : **Petersen, Uwe, Dr.**
**Auf dem Forst 4**
**D-5090 Leverkusen 1 (DE)**
Erfinder : **Stadler, Peter, Dr.**
**Ohligserstrasse 85**
**D-5657 Haan (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 17, rue du Louvre, 75001 Paris, France

Selektiv geschützte 4,6-Di-O-(Aminoglykosyl)-1,3-diamino-cyclitole, Verfahren zu deren Herstellung und deren Verwendung als Zwischenprodukte

Die Erfindung betrifft neue, selektiv geschützte 4,6-Di-O-(aminoglykosyl)-1,3-diaminocyclitole, die als Zwischenprodukte zur Herstellung wertvoller neuer und bekannter Antibiotika dienen, sowie ein Verfahren zur Herstellung der selektiv geschützten Verbindungen.

Die neuen Verbindungen lassen sich durch die Formel (I)

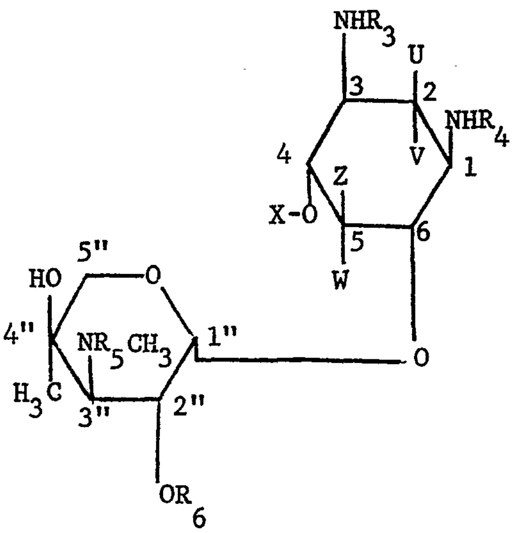

(I)

in der

X für einen Rest der Formeln

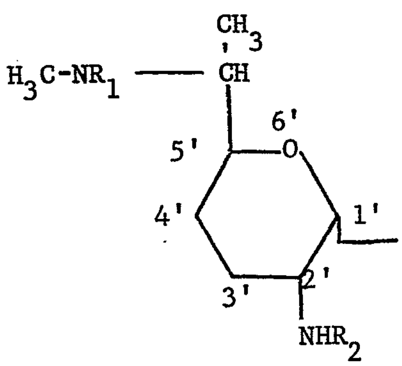

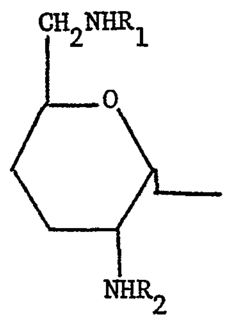

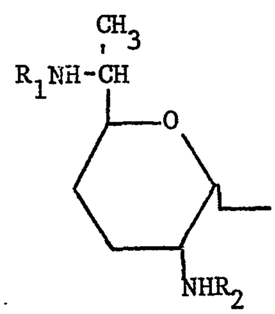

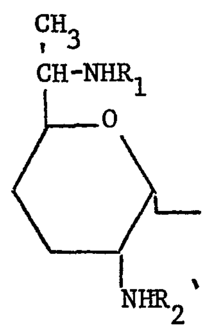

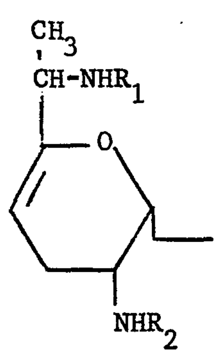

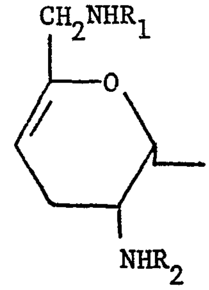

U, V, W und Z für Wasserstoff oder Hydroxy,
$R_1$, $R_2$ und $R_3$ für $-CO-A$,
$R_4$ für Wasserstoff,
$R_5$ für einen Rest $-SR_7$ und
$R_6$ für Wasserstoff oder einen abspaltbaren, raumerfüllenden Substituenten stehen,
wobei U und V sowie W und Z nicht gleichzeitig OH und wobei A einen Rest der Formeln

$-CHal_3$, $-(CH_2)_nB$, $-O-E$, 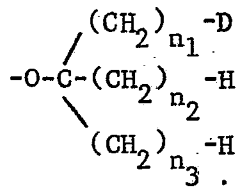 oder 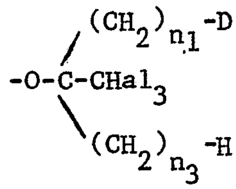

B und D Wasserstoff oder gegebenenfalls substituiertes Phenyl,
E gegebenenfalls substituiertes Phenyl,
n eine Zahl von 0-5,
$n_1$, $n_2$ und $n_3$ eine Zahl von 0-5,
Hal Fluor, Chlor oder Brom und
$R_7$ gegebenenfalls substituiertes Phenyl, Di- oder Triphenylmethyl
bedeuten, wiedergegeben.

Geeignete Substituenten der gegebenenfalls substituierten Phenyl-, Di- oder Triphenylmethylreste $R_7$ sind z.B. 1 bis 3 Substituenten aus der Reihe Trifluormethyl, Nitro, $C_1$-$C_4$-Alkyl, $C_1$-$C_4$-Alkoxy, $C_1$-$C_4$-Alkoxycarbonyl oder Phenyl oder 1 bis 5 Halogenatome, vorzugsweise Chloratome. Als Beispiel für $-SR_7$-Gruppen seien o-Nitrophenylsulfenyl und 2,4,5-Trichlorphenylsulfenyl genannt.

Geeignete Substituenten der gegebenenfalls substituierten Phenylreste B, D und E sind 1 oder 2 Substituenten aus der Reihe Nitro, Halogen, vorzugsweise Chlor, $C_1$-$C_4$-Alkyl, $C_1$-$C_4$-Alkoxy und Phenyl.

Raumerfüllende, abspaltbare Reste $R_6$ sind beispielsweise solche der Formeln

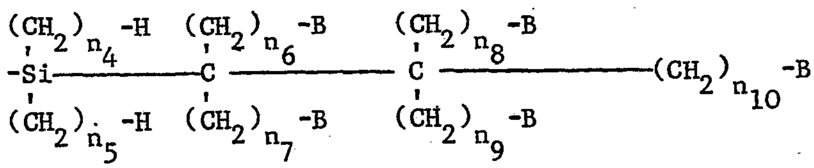 oder

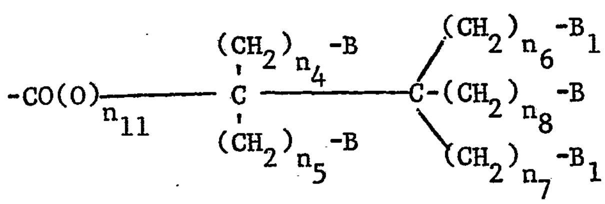

worin
$n_4$, $n_5$, $n_6$, $n_7$, $n_8$, $n_9$, $n_{10}$ eine Zahl 0-3
$n_{11}$ eine Zahl 0 oder 1,
$B_1$ B, $C_1$-$C_5$-Alkoxy oder $C_3$-$C_5$-Alkenyloxy bedeuten und
B die vorstehend genannte Bedeutung aufweist.

Die folgenden Silylgruppen sind besonders bevorzugt:
Dimethyl-(1,2-dimethylpropyl)-silyl, Dimethyl-(2,4,4-trimethylpentyl)-silyl, Dimethyl-(1,1,2-trimethylpropyl)-silyl, Dimethyl-(2-methyl-2-phenylethyl)-silyl, Dimethyl-(1,1,4,4-tetramethylbutyl)-silyl und Dimethyl-[2-methyl-2-(4-methyl-cyclohex-3-enyl)-ethyl]-silyl.

Von besonderem Interesse sind die erfindungsgemäßen N-geschützten Verbindungen gemäß Formel (I), die sich von den Antibiotika Gentamicin $C_1$, Gentamicin $C_{1a}$, Gentamicin $C_2$, Gentamicin $C_{2a}$, Gentamicin $C_{2b}$, Sisomicin, Verdamicin, G 52, Mutamicin 1, Mutamicin 2, Mutamicin 4 und Mutamicin 6 ableiten.

Von diesen sind die durch die Formel (II) wiedergegebenen Derivate von Sisomicin

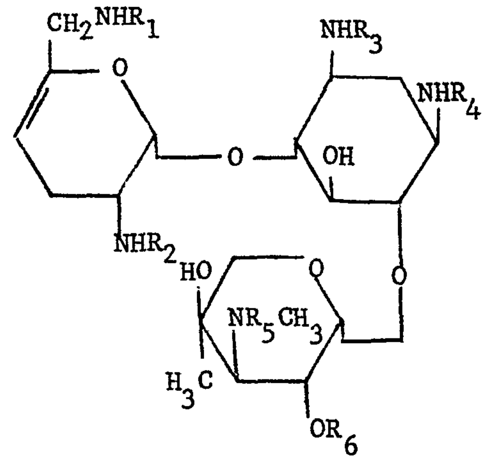

(II)

worin

$R_1$, $R_2$, $R_3$, $R_4$, $R_5$ und $R_6$ die oben angegebene Bedeutung haben,

besonders wertvoll.

Die erfindungsgemäßen Verbindungen der Formel (I), werden erhalten, indem man Verbindungen der Formel (I), in denen

$R_1$, $R_2$, $R_3$, $R_4$ und $R_5$ eine Gruppe $-SR_7$ und

$R_6$ Wasserstoff bedeuten und

U, V, W, Z und $R_7$ die bereits bekannte Bedeutung haben,

1. mit einem Silylierungsmittel oder einem Acylierungsmittel der Formel

$$R_6-G \qquad (III)$$

worin

$R_6$ mit Ausnahme von Wasserstoff die bereits genannte Bedeutung hat, und

G eine Abgangsgruppe bedeutet,

umsetzt,

2. die $-S-R_7$-Gruppen $R_1$, $R_2$, $R_3$ und gegebenenfalls $R_4$ abspaltet,

3. die freigesetzten Aminogruppen mit Ausnahme der 1-$NH_2$-Gruppe, falls diese im Schritt 2. bereits freigesetzt wurde, mit Acylierungsmitteln der Formel

$$A-CO-G \qquad (IV)$$

worin

A die bereits genannte Bedeutung hat und

G eine Abgangsgruppe bedeutet,

acyliert,

4. falls nicht unter 2. geschehen, die $S-R_7$-Gruppe $R_4$ abspaltet und gegebenenfalls,

5. den raumerfüllenden Substituenten $R_6$ abspaltet, wobei die Schritte 4. und 5. in ihrer Reihenfolge auch vertauscht werden können.

Geeignete Abgangsgruppen G sind bei der Silylierung beispielsweise Halogen, insbesondere Chlor und Perfluorbutylsulfonyloxy ; bei der Acylierung beispielsweise Halogen, ein aktivierender Esterrest wie p-Nitrophenoxy oder ein Rest A—CO—O, wobei A die bereits genannte Bedeutung hat.

Die Silylierung wird in einem inerten Lösungsmittel wie Dichlormethan oder Chloroform in Gegenwart eines Katalysators, der zugleich die zur Bindung der freiwerdenden Säure benötigte Hilfsbase darstellt, durchgeführt. Dafür sind alle organischen Basen, die die Si−G-Bindung aktivieren, geeignet. Vorzugsweise wird mit äquivalenten Mengen Imidazol als Katalysatorbase gearbeitet. Silylierungsmittel und Katalysatorbase können in jeweils stöchiometrischen Mengen mit der Verbindung der Formel (I), in der $R_1$, $R_2$, $R_3$, $R_4$ und $R_5$ $-S-R_7$ bedeuten, umgesetzt werden, jedoch werden erheblich kürzere Reaktionszeiten mit einem 2-5-fachen Überschuß an Silylierungsmittel erzielt. Die Reaktionstemperatur beträgt 0-80 °C, vorzugsweise 20-40 °C.

Die Acylierung im Schritt 1 wird bei Verwendung von Säureanhydriden oder aktivierten Estern in basischen Lösungsmitteln, vorzugsweise in Pyridin mit einem 1-10-fachen, vorzugsweise 3-5-fachen Überschuß an Acylierungsmittel in Temperaturbereich von 0-80 °C, vorzugsweise 10-30 °C durchgeführt. Bei der Verwendung von Säurehalogeniden als Acylierungsmitteln werden diese in stöchiometrischen

Mengen oder in einem 2-3-fachen Überschuß in einem inerten Lösungsmittel wie Dichlormethan in Abwesenheit von äquivalenten Mengen einer organischen Hilfsbase, beispielsweise Triäthylamin, bei 0-40 °C, vorzugsweise bei 10-30 °C umgesetzt. Die Reaktionszeiten werden wesentlich durch Zusatz von 10-20 Mol-% eines Katalysators wie p-Dimethylaminopyridin oder p-Pyrrolidino-pyridin herabgesetzt.

Die Abspaltung der Schutzgruppen $S-R_7$ erfolgt im allgemeinen durch Reaktion mit Nucleophilen, vorzugsweise mit H—S-Gruppen-haltigen Nikleophilen wie $H_2S$, Thiophenol oder 2-Mercaptobenzthiazol bei 0-20 °C in einem geeigneten organischen Lösungsmittel wie Dichlormethan, Methanol oder in Gemischen solcher Lösungsmittel.

Die Menge des einzusetzenden Abspaltungsmittels richtet sich nach dessen Reaktivität, meist wird es im 2-5-fachen Überschuß eingesetzt. Die Abspaltung der $S-R_7$-Gruppen $R_1$, $R_2$ und $R_3$ ohne die Abspaltung der $S-R_7$-Gruppe $R_4$ und $R_5$ kann leicht dadurch erzielt werden, indem man zu kürzeren Reaktionszeiten und/oder niedrigeren Abspaltungstemperaturen und/oder geringerem Nukleophil-Überschuß übergeht.

Die Einführung der Acylgruppen CO—A erfolgt nach allgemein üblichen Methoden wie sie beispielsweise in Houben-Weyl, Methoden der organischen Chemie, Band XV, Georg Thieme-Verlag, Stuttgart 1974, beschrieben sind. Die Auswahl der Acylgruppen CO—A hängt davon ab, für welche weitere Reaktionen die erfindungsgemäßen Zwischenprodukte eingesetzt werden sollen.

Wurde die $S-R_7$-Schutzgruppe $R_4$ bereits im zweiten Reaktionsschritt abgespalten, so muß die Einführung der Acylgruppen $R_1$, $R_2$ und $R_3$ selektiv erfolgen, so daß die 1-$NH_2$-Gruppe, die unter dem sterischen Einfluß des raumerfüllenden Substituenten $R_6$ abgeschirmt ist, nicht angegriffen wird. Hierbei werden z.B. die reaktiven Ester der Säuren A—COOH wie die p-Nitrophenylester oder die N-Hydroxysuccinimidester mit Erfolg verwendet.

Die Abspaltung der Schutzgruppe $R_6$ kann entweder in mit Wasser nicht mischbaren organischen Lösungsmitteln wie Dichlormethan mit Tetralkylammoniumfluoriden, z.B. Tetrabutylammoniumfluorid oder aber in Dimethylsulfoxid-Wasser-Gemischen entweder mit anorganischen Fluoriden wie Kaliumfluorid oder mit anorganischen Basen wie Natronlauge erfolgen, vorzugsweise bei Raumtemperatur.

Die erfindungsgemäßen Zwischenprodukte der Formel (I) dienen zur Darstellung von 4,6-Di-O-(aminoglykosyl)-1,3-diaminocyclitolen, welche an der 1-$NH_2$-Gruppe substituiert sind, insbesondere von Derivaten der Antibiotika Gentamicin $C_1$, Gentamicin $C_{1a}$, Gentamicin $C_2$, Gentamicin $C_{2a}$, Gentamicin $C_{2b}$, Sisomicin, Verdamicin, G 52, Mutamicin 1, Mutamicin 2, Mutamicin 4 und Mutamicin 6.

Diese Antibiotika sind wertvolle Substanzen zur wirkungsvollen Bekämpfung bakterieller Infektionen. Ihre hohe Wirksamkeit ist jedoch häufig verbunden mit relativ großer Nephro- und Ototoxizität ; hinzu kommt Resistenzbildung der bekämpften Keime. Aus diesen Gründen ist es wünschenswert, Derivate der Aminoglykosid-Antibiotika mit verbesserten Eingenschaften herzustellen, die bei verringerter Toxizität eventuell auch die Bekämpfung resistenter Keime ermöglichen. Als Substanzen mit derartig verbesserten Eigenschaften sind verbindungen wie 1-N-Acetylsisomicin und 1-N-Äthylsisomicin bekannt geworden (DT-A 2 437 160).

Die Darstellung mono-N-substituierter Sisomicin-Derivate erweist sich jedoch ausgehend von ungeschütztem Sisomicin als schwierig, da im Sisomicin-Molekül fünf Aminogruppen vergleichbarer Reaktivität vorhanden sind. Man gelangt daher immer zu Reaktionsgemischen, welche chromatografisch aufgetrennt werden müssen. Ein Beispiel hierfür sind die in FR-A 2 240 015 (Seite 45, Zeile 22 bis 23) beschriebenen Sisomicin-Derivate, welche nicht selektiv herstellbar sind, sondern aus einem Gemisch ihrer Isomeren auf chromatografischem Wege isoliert werden. Bei technischen Ansätzen sind jedoch derartige Trennungsverfahren nicht brauchbar bzw. sehr kostenaufwendig.

Das erfindungsgemäße Verfahren liefert demgegenüber selektiv in der 1-Stellung eine freie Aminogruppe aufweisende, an den übrigen Aminogruppen blockierte Aminoglycoside in isomerenfreier Form. Die Natur der Schutzgruppen ist dabei so beschaffen, daß sowohl Alkylierungen als auch Acylierungen an den freien Stickstoffatomen sowie eine anschließende schonende Entblockierung möglich sind.

Die Sulfenyl-Schutzgruppen haben im erfindungsgemäßen Verfahren außerdem die unerwartete vorteilhafte Wirkung, daß sie selektiv an den Positionen 2', 3 und 6' abgespalten werden können. Ihre Verwendung ermöglicht es daher, nach der Re-Acylierung der Aminogruppen in 2', 3 und 6'-Position und der Entfernung von $R_6$ auch noch die Schutzgruppe an der Aminogruppen in 1-Stellung zu entfernen, ohne daß die Schutzgruppe in der 3''-N-Position mit abgespalten wurd, was weitere interessante Derivatisierungsmöglichkeiten erörffnet.

Ein weiterer Vorteil des erfindungsgemäßen Verfahrens besteht darin, daß während des gesamten Synthesewegs eine chromatographische Reinigungsoperation der Zwischenprodukte nicht erforderlich ist, und die Synthese somit auch im technischen Maßstab anwendbar ist.

## Beispiel 1

Penta-N-(o-nitrophenylsulfenyl)-sisomicin

166,1 g Sisomicinsulfat in 300 ml 4 n NaOH und 1 350 ml Dioxan werden gleichzeitig so mit einer Lösung von 240 g o-Nitrophenylsulfenylchlorid in 900 ml Dioxan und mit 390 ml 4 n NaOH versetzt, daß

ein pH von 12-14 eingehalten wird. Der Niederschlag wird abgesaugt und verworfen, das Filtrat in 5 l Wasser eingerührt, der gelbe amorphe Niederschlag abgesaugt, mit 150 ml Methanol gewaschen und getrocknet. Ausbeute 290 g (100 % der Theorie) rohes Produkt, das ohne weitere Reinigung für die weiteren Umsetzungen eingesetzt wird. Ein reines Präparat kann nach Chromatographie an Kieselgel mit einem Laufmittelgemisch $CH_2Cl_2/CH_3OH$ = 97,5/2,5 erhalten werden.

$R_F$ = 0,62 ($CH_2CH_2/CH_3OH$ = 97,5/2,5) ;
13-C-NMR ($CDCl_3$) :
δ = 124-148 (arom. C) ;
102,3 (C-1″) ; 99,0 (C-1′) ;
97,92 (C-4′) ; 89,05 (C-6) ;
82,33 (C-4) ; 53,31 (C-1) ;
56,73 (C-3) ppm.

## Beispiel 2

Penta-N-(o-nitrophenylsulfenyl)-2″-O-[dimethyl-(1.2-dimethylpropyl)-silyl]-sisomicin

60,6 g rohes Penta-N-(o-nitrophenylsulfenyl)-sisomicin und 8,75 g Imidazol werden in 250 ml absolutem Dichlormethan gelöst. Bei 0 °C werden unter Feuchtigkeitsausschluß 22,5 ml Dimethyl-(1,2-dimethylpropyl)-silylchlorid zugetropft. Der Ansatz wird im Vakuum auf ca. 170 ml eingedampft und bei Raumtemperatur 48 Stunden stehen gelassen. Nach Zugabe von 130 ml absolutem Dichlormethan wird der Niederschlag abgesaugt, daß Filtrat mit 350 ml Petroläther kräftig durchgeschüttelt und die Petrolätherphase abdekantiert und verworfen. Das ausgefallene Öl wird in 100 ml Dichlormethan gelöst, mit 250 ml Petroläther erneut ausgefällt und schließlich im Hochvakuum getrocknet. Ausbeute 60 g (89 %) Rohprodukt, das ohne weitere Reinigung für die weiteren Umsetzungen eingesetzt wird. Ein reines Präparat wird durch Chromatographie an Kieselgel mit $CH_2Cl_2/CH_3OH$ = 99/1 erhalten.

$R_F$ ($CH_2Cl_2/CH_3OH$ = 99,5/0,5) : 0,62 ;
13-C-NMR ($CDCl_3$) :
δ = 124-138 (arom. C) ;
147,54 (C-5′) ; 102,26 (C-1″) ;
97,81 (C-4′) ; 99,09 (C-1′) ;
− 2,9 bis − 3,0 (Si-$CH_3$) ;
22,77 (Si−CH−) ; 30,60 (Si−CH−CH) ppm.

Als Nebenprodukt wird das Penta-N-(o-nitrophenylsulfenyl)-2″, 5- bis -O-[dimethyl-(1,2-dimethylpropyl)-silyl]-sisomicin isoliert.

$R_F$ ($CH_2Cl_2/CH_3OH$ = 99,5/0,5) : 0,79 ;
13-C-NMR ($CDCl_3$) :
δ = 124-146 (arom. C),
148,00 (C-5′) ; 96,13 (C-4′) ppm.

## Beispiel 3

Penta-N-(o-nitrophenylsulfenyl)-2″-O-(2-äthylhexanoyl)-sisomicin

60,6 g rohes Penta-N-(o-nitrophenylsulfenyl)-sisomicin in 250 ml absolutem Dichlormethan werden mit 15 ml Triäthylamin und 1,5 g p-Dimethylaminopyridin versetzt. Dazu werden bei 0 °C 13 ml 2-Äthylhexanoylchlorid in 25 ml absolutem Dichlormethan getropft. Nach 20 Stunden bei Raumtemperatur wird der Ansatz zweimal mit je 50 ml $H_2O$ ausgeschüttelt, die organische Phase mit $Na_2SO_4$ getrocknet und das Produkt durch Ausfällen mit Petroläther isoliert. Die Ausbeute an Rohprodukt nach dem Trocknen im Hochvakuum ist quantitativ.

Ein reines Präparat wird durch Chromatographie an Kieselgel mit $CH_2Cl_2/CH_3OH$ = 99/1 erhalten.

$R_F$ ($CH_2Cl_2/CH_3OH$ = 99,5/0,5) : 0,41 ;
13-C-NMR ($CDCl_3$) :
δ = 124-146 (arom. C) ; 175,77 (CO) ;
53,22 (CO−CH−) ; 99,73 (C-1″) ;
99,13 (C-1′) ; 97,95 (C-4′) ; 147,75 (C-5′) ;
82,13 (C-4) ; 76,47 (C-5), 88,98 (C-6) ;
69,30 (C-2″) ; 70,89 (C-4″) ; 68,06 (C-5″) ppm.

## Beispiel 4

Penta-N-(o-nitrophenylsulfenyl)-2″-O-butyryl-sisomicin

10 g rohes Penta-N-(o-nitrophenylsulfenyl)-sisomicin werden mit 33 ml Pyridin und 60 ml Buttersäureanhydrid 48 Stunden bei 20 °C stehen gelassen, im Hochvakuum eingedampft, der Rückstand in Dichlormethan aufgenommen und mit Petroläther ausgefällt. Die Ausbeute an Rohprodukt ist quantitativ. Ein reines Präparat wird durch Chromatographie mit $CH_2Cl_2/CH_3OH$ = 99/1 an Kieselgel erhalten :

$R_F$ ($CH_2Cl_2/CH_3OH$ = 99/1) : 0,46 ;
13-C-NMR ($CDCl_3$) :
δ = 172,94 (CO) ; 147,82 (C-5') ; 99,96 (C-1") ;
99,19 (C-1') ; 98,03 (C-4') ppm.

Als Nebenprodukt wird das Penta-N-(o-nitrophenylsulfenyl)-2", 5- bis -O-butyryl-sisomicin isoliert.

$R_F$ ($CH_2Cl_2/CH_3OH$ = 99/1) : 0,65 ;
13-C-NMR ($CDCl_3$) :
δ = 172,30 und 173,27 (jeweils CO) ; 148,07 (C-5') ;
100,46 (C-1") ; 98,42 (C-1') ; 98,02 (C-4') ppm.

## Beispiel 5

Penta-N-(o-nitrophenylsulfenyl)-2"-O-isovaleryl-sisomicin

24,24 g Penta-N-(o-nitrophenylsulfenyl)-sisomicin in 100 ml absolutem Dichlormethan werden mit 7,2 ml Triäthylamin und 720 mg 4-Dimethylaminopyridin versetzt. Bei 0 °C werden 4,8 ml Isovalerylchlorid in 5 ml Dichlormethan zugetroft. Nach 20 Stunden bei Raumtemperatur wird der Ansatz analog Beispiel 3 aufgearbeitet, Quantitative Ausbeute an Rohprodukt.

$R_F$ ($CH_2Cl_2/CH_3OH$ = 99/1) : 0,46.

## Beispiel 6

1,3"-Bis-N-(o-nitrophenylsulfenyl)-2"-O-[dimethyl-(1,2-dimethyl-propyl)-silyl]-sisomicin

56 g rohes Penta-N-(o-nitrophenylsulfenyl)-2"-O-[dimethyl-(1,2-dimethyl-propyl)-silyl]-sisomicin in 36 ml Dichlormethan/70 ml Methanol werden mit 16 g 2-Mercapto-benthiazol versetzt, geschüttelt, bis klare Lösung erfolgt und bei 5 °C 2 Stunden stehen gelassen. Der sich dabei abscheidende Niederschlag wird abfiltriert und die Lösung ohne Isolierung des gewünschten Produktes für die weiteren Umsetzungen verwendet. Die Ausbeute beträgt etwa 80 % der Theorie. Zur Darstellung eines reinen Präparats wird das Filtrat rasch im Vakuum eingedampft und der Rückstand mit a) Dichlormethan, b) Dichlormethan/$CH_3OH$ (8:2) und c) mit $CH_2Cl_2/CH_3OH$/20 % wäßrigem Ammoniak (7:2,7:0,3), an Kieselgel chromatographiert. Die Ausbeute an reinem Produkt beträgt 25,3 g (69 %).

$R_F$ ($CH_2Cl_2/CH_3OH$/20 % wäßriges $NH_3$ = 7 : 2,7 : 0,3) = 0,66 ;
13-C-NMR ($CD_3OD$) :
δ = 1,5 ($Si-CH_3$) ; 122-146 (arom. C) ;
147,14 (C-5') ; 103,31 (C-1") ; 100,16 (C-1') ;
99,30 (C-4') ppm.

Als Nebenprodukt werden bei der Säulenchromatographie 3 g (10 %) 3"-N-(o-Nitrophenylsulfenyl)-2"-O-[dimethyl-(1,2-dimethyl-propyl)-silyl]-sisomicin isoliert.

$R_F$ ($CH_2Cl_2/CH_3OH$/20 % wäßriges $NH_3$ = 7 : 2,7 : 0,3) = 0,15 ;
13-C-NMR ($CD_3OD$) :
δ = 76,66 (C-2") ; 21,70 (C-6") ; 30,40 ($N-CH_3$) ;
53,13 (C-1), 52,18 (C-3) ; 44,06 (C-6') ;
49,41 (C-2') ppm.

## Beispiel 7

1,3"-Bis-N-(o-nitrophenylsulfenyl)-2"-O-butyryl-sisomicin

10 g rohes Penta-N-(o-nitrophenylsulfenyl)-2"-O-butyryl-sisomicin in 117 ml Dichlormethan/58 ml Methanol werden mit 7 g 2-Mercaptobenzthiazol geschüttelt, bis eine klare Lösung entstanden ist und 2 Tage bei 5 °C stehen gelassen. Nach Abfiltrieren des Niederschlags wird das Filtrat ohne Isolierung des gewünschten Produktes weiterverarbeitet.

$R_F$ ($CH_2Cl_2/CH_3OH$/20 % wäßriges $NH_3$ 3,5 : 1,35 : 0,15) = 0,45.

## Beispiel 8

1,3"-Bis-N-(o-nitrophenylsulfenyl)-2"-O-isovaleryl-sisomicin

48 g rohes Penta-N-(o-nitrophenylsulfenyl)-2"-O-isovaleryl-sisomicin werden mit 120 ml Dichlormethan, 120 ml Methanol und 27 g 2-Mercaptobenzthiazol geschüttelt, bis eine klare Lösung entstanden ist und dann 2 Stunden bei 5 °C stehen gelassen. Der Niederschlag wird abgesaugt, das Filtrat rasch im Vakuum eingedampft, der Rückstand in Dichlormethan aufgenommen, die Lösung zweimal mit Wasser ausgeschüttelt, über $Na_2SO_4$ getrocknet, das Produkt mit Äther ausgefällt und der Niederschlag im Vakuum getrocknet. Rohausbeute 23 g (74 %). Eine gereinigte Substanz wird durch Chromatographie an Kieselgel nach den für Beispiel 6 beschriebenen Methoden erhalten.

$R_F$ (Laufmittel wie Beispiel 7) = 0,46.

## Beispiel 9

1,3″-Bis-N-(o-nitrophenylsulfenyl)-2″-O-(2-äthylhexanoyl)-sisomicin

16 g rohes Penta-N-(o-nitrophenylsulfenyl)-2″-O-(2-äthylhexanoyl)-sisomicin werden mit 40 ml Dichlormethan, 40 ml $CH_3OH$ und 9 g 2-Mercaptobenzthiazol geschüttelt, bis eine klare Lösung entstanden ist, die 7 Stunden bei 5 °C stehen gelassen wird. Der Niederschlag wird abfiltriert und das Filtrat rasch im Vakuum eingedampft. Die Reinigung erfolgt analog Beispiel 6 durch Chromatographie an Kieselgel. Die Ausbeute beträgt 6,6 g (63 % der Theorie).

$R_F$ (Laufmittel wie Beispiel 7) = 0,58 ;
13-C-NMR ($CD_3OD$) :
δ = 177,13 (CO), 151,14 (C-5′) ; 124-146 (arom. C) ;
101,06 (C-1′ und C-1″) ; 96,57 (C-4′) ; 71,17 (C-2″) ppm.

## Beispiel 10

1,3″-Bis-N-(onitrophenylsulfenyl)-2′,3,6′-triacetyl-2″-O-[dimethyl-(1,2-dimethyl-propyl)-silyl]-sisomicin

Die rohe Lösung von 1,3″-Bis-N-(o-nitrophenylsulfenyl)-2″-O-[dimethyl-(1,2-dimethyl-propyl)-silyl]-sisomicin wird innerhalb von 2 Minuten unter Eiskühlung mit 15 ml Acetanhydrid und 38 ml 6 n NaOH so versetzt, daß das Reaktionsgemisch stets alkalisch bleibt. Der Ansatz wird im Vakuum eingedampft, bis das rote Öl sich klar absetzt. Die wäßrige Phase wird abdekantiert und das Öl mit 120 ml $H_2O$ im Vakuum bei 30-40 °C so durchgerührt, daß ein Teil (ca. 20-30 ml) des Wassers abdestilliert. Die Wasserphase wird abdekantiert und das Öl ohne Reinigung weiterverarbeitet. Chromatographie an Kieselgel mit $CH_2Cl_2/CH_3OH$ = 95/5 liefert ein reines Präparat.

$R_F$ ($CH_2Cl_2/CH_3OH$ = 95/5) : 0,18 ;
($CH_2Cl_2/CH_3OH$ = 90/10) : 0,7 ;
H-NMR (220 MHz) :
δ = 2,02, 1,92, 1,89 ($CH_3-CO$) ;
3,03 ($N-CH_3$) ppm.

## Beispiel 11

1,3″-Bis-N-(o-nitrophenylsulfenyl)-2′,3,6′-tris-(tert.-butoxycarbonyl)-2″-O-[dimethyl-(1,2-dimethyl-propyl)-silyl]-sisomicin

Die rohe Lösung von 1,3″-Bis-N-(o-nitrophenylsulfenyl)-2″-O-[dimethyl-(1,2-dimethylpropyl)-silyl]-sisomicin wird mit 175 ml basischem Ionenaustauscher ($OH^{\ominus}$-Form, mit Methanol gewaschen) kräftig gerührt und der Ansatz mit 35 ml tert.-Butyl-pyrocarbonat versetzt. Nach 30 Minuten bei Raumtemperatur wird der Ansatz eingedampft und ohne weitere Reinigung weiterverarbeitet. Ein reines Präparat wird durch Chromatographie an Kieselgel (Laufmittel $CH_2Cl_2/CH_3OH$ = 99/1) erhalten.

$R_F$ ($CH_2Cl_2/CH_3OH$ = 98,5/1,5) : 0,31 ;
($CH_2Cl_2/CH_3OH$ = 97,5/2,5) : 0,68 ;
13-C-NMR ($CDCl_3$) :
δ = 102,51 (C-1″) ; 55,33 (C-1) ; 49,28 (C-3) ;
89,03 (C-6) ; 97,43 (C-1′) ; 47,13 (C-2′) ;
150,00 (C-5′) ; 43,00 (C-6′) ;
155,43 (Co) ; 79,66 [$(CH_3)_3\underline{\underline{C}}$] ; 28,50 [$(\underline{\underline{C}}H_3)_3$-C] ;
27,91 ($\underline{\underline{C}}H-Si$) ppm.

## Beispiel 12

1,3″-Bis-N-(o-nitrophenylsulfenyl)-2′,3,6′-tris-(1,1-dimethyl-2,2,2-trichlor-ethoxycarbonyl)-2″-O-[dimethyl-(1,2-dimethyl-propyl)-silyl]-sisomicin

Die rohe Lösung von 1,3″-Bis-N-(o-nitrophenylsulfenyl)-2″-O-[dimethyl-(1,2-dimethyl-propyl)-silyl]-sisomicin wird mit 18 ml 6 n NaOH versetzt und das Methanol im Vakuum abgedampft. Der Rückstand wird mit 87 ml Dichlormethan und 78 ml 2 n NaOH und bei 0 °C tropfenweise mit 38 ml 1,1-Dimethyl-2,2,2-trichlor-ethyl-oxycarbonylchlorid innerhalb von 5 Minuten versetzt. Nach Zugabe von 350 ml $H_2O$ wird die organische Phase abgetrennt, die Wasserphase mit 350 ml Dichlormethan extrahiert und die vereinigten organischen Extrakte im Vakuum eingedampft. Das in quantitativer Rohausbeute anfallende Öl wird ohne Reinigung weiterverarbeitet. Eine reine Probe wird durch Chromatographie an Kieselgel (Laufmittel $CH_2Cl_2/CH_3OH$ = 99,5/0,5) erhalten.

$R_F$ ($CH_2Cl_2/CH_3OH$ = 99,5/0,5) : 0,45 ;
13-C-NMR ($CDCl_3$) :
δ = 102,59 (C-1″) ; 55,63 (C-1) ; 50,38 (C-3) ;
79,50 (C-4) ; 88,96 (C-6) ; 47,42 (C-2′) ;
97,35 (C-4′) ; 146,33 (C-5′) ; 43,00 (C-6′) ppm.

Beispiel 13

1,3″-Bis-N-(o-nitrophenylsulfenyl)-2′,3,6′-tris-N-(2,2,2-trichlorethoxycarbonyl)-2″-O-[dimethyl-propyl)-silyl]-sisomicin

8,8 g 1,3″-Bis-N-(o-nitrophenylsulfenyl)-2″-O-[dimethyl-(1,2-dimethyl-propyl)-silyl]-sisomicin in 20 ml Dichlormethan werden mit 24 ml 2 n NaOH kräftig gerührt und bei 0 °C mit 5 ml (2,2,2-Trichloräthyl)-oxycarbonylchlorid tropfenweise versetzt. Der Ansatz wird mit 20 ml Dichlormethan und 20 ml $H_2O$ verdünnt, die Wasserphase abgetrennt, die organische Phase noch zweimal mit je 10 ml Wasser gewaschen, über $Na_2SO_4$ getrocknet und im Vakuum eingedampft. Ausbeute 13,8 g (98 %).

$R_F$ ($CH_2Cl_2/CH_3OH$ = 99/1) : 0,34.

Beispiel 14

1,3″-Bis-N-(o-nitrophenylsulfenyl)-2′,3,6′-tris-N-trichloracetyl-2″-O-[dimethyl-(1,2-dimethyl-propyl)-silyl]-sisomicin

8,8 g 1,3″-Bis-N-(o-nitrophenylsulfenyl)-2″-O-[dimethyl-(1,2-dimethyl-propyl)-silyl]-sisomicin in 20 ml Dichlormethan/20 ml Pyridin werden bei −15 °C tropfenweise mit 7,5 ml Trichloressigsäureanhydrid versetzt und noch 10 Minuten bei Raumtemperatur weitergerührt. Nach Zugabe von 20 ml Dichlormethan wird der Ansatz zweimal mit je 20 ml $H_2O$ ausgeschüttelt, die organische Phase eingedampft und der Rückstand als Rohprodukt weiterverarbeitet.

$R_F$ ($CH_2Cl_2/CH_3OH$ = 97,5/2,5) = 0,72.

Beispiel 15

1,3″-Bis-N-(o-nitrophenylsulfenyl)-2′,3,6′-tris-(4-methoxybenzyloxycarbonyl)-2″-O-[dimethyl-(1,2-dimethylpropyl)-silyl]-sisomicin

3,52 g 1,3″-Bis-N-(o-nitrophenylsulfenyl)-2″-O-[dimethyl-(1,2-dimethyl-propyl)-silyl]-sisomicin in 10 ml Dichlormethan werden mit 14 ml 2 n NaOH verrührt und bei 0 °C mit 4 ml frisch dargestelltem 4-Methoxybenzyloxycarbonylchlorid versetzt. Der Ansatz wird mit 10 ml Dichlormethan verdünnt, die Wasserphase abgetrennt, die organische Phase noch zweimal mit je 10 ml Wasser gewaschen, über $Na_2SO_4$ getrocknet und im Vakuum eingedampft. Die Rohausbeute beträgt 5 g (91 % der Theorie). Das Produkt wird ohne Reinigung weiterverarbeitet. Ein reines Präparat wird durch Chromatographie an Kieselgel (Laufmittel $CH_2Cl/CH_3OH$ = 99/1) erhalten.

$R_F$ ($CH_2Cl_2/CH_3OH$ = 98,5/1,5) = 0,37.

Beispiel 16

1,3″-Bis-N-(o-nitrophenylsulfenyl)-2′,3,6′-tris-N-phenoxycarbonyl-2″-O-[dimethyl-(1,2-dimethyl-propyl)-silyl]-sisomicin

8,8 g 1,3″-Bis-N-(o-nitrophenylsulfenyl)-2″-O-[dimethyl-(1,2-dimethyl-propyl)-silyl]-sisomicin werden in 20 ml Dichlormethan gelöst und mit 17 ml 2 n NaOH versetzt. Zu dieser Emulsion werden während 3 bis 4 Minuten bei 0 °C 4,7 g Chlorameisensäurephenylester getropft. Die Aufarbeitung erfolgt wie in Beispiel 15. Das Rohprodukt wird ohne weitere Reinigung weiterverarbeitet.

$R_F$ ($CH_2Cl_2/CH_3OH$ = 97,5/2,5) : 0,62.

Beispiel 17

1,3″-Bis-N-(o-nitrophenylsulfenyl)-2′,3,6′-N-triacetyl-2″-O-isovaleryl-sisomicin

10 g rohes 1,3″-Bis-N-(o-nitrophenylsulfenyl)-2″-O-isovaleryl-sisomicin in 30 ml $CH_2Cl_2$/20 ml $CH_3OH$

werden unter Eiskühlung mit 20 ml Acetanhydrid versetzt, nach 10 Minuten im Vakuum eingedampft und an einer Kieselgelsäule (2,5 × 10 cm, Laufmittel $CH_2Cl_2/CH_3OH$ = 95/5) chromatographiert. Die Ausbeute beträgt 5,9 g (51 % der Theorie).

$R_F$ ($CH_2Cl_2/CH_3OH$ = 9/1) : 0,68 ;
13-C-NMR ($CDCl_3$) :
δ = 41,50 ($N-CH_3$) ; 70,47 (C-2″) ;
55,16 (C-1) ; 50,26 (C-3) ; 80,04 (C-4) ;
89,01 (C-6) ; 47,32 (C-2′) ; 43,13 (C-6′) ;
96,23 (C-1′) ; 171,14, 170,26 (drei CO-Gruppen) ;
172,04 ($O-\underset{\underset{O}{\|}}{C}-$) ppm.

## Beispiel 18

1,3″-Bis-N-(o-nitrophenylsulfenyl)-2′,3,6′-N-triacetyl-2″-O-(2-ethyl-hexanoyl)-sisomicin

8 g rohes Penta-N-(o-nitrophenylsulfenyl)-2″-O-(2-ethylhexanoyl)-sisomicin werden wie in Beispiel 9 beschrieben zu 1,3″-Bis-N-(o-nitrophenylsulfenyl)-2″-O-(2-ethylhexanoyl)-sisomicin umgesetzt, der ausgefallene Niederschlag abgesaugt und zweimal mit je 7,5 ml Methanol gewaschen. Das Filtrat wird mit 5 ml Acetanhydrid in Gegenwart von 17,5 ml 4 n NaOH unter Eiskühlung umgesetzt und der Ansatz wie für Beispiel 10 beschrieben aufgearbeitet. Das resultierende rote Öl wird im Vakuum getrocknet und ist für die Weiterverarbeitung genügend rein. Die Ausbeute ist 6 g (100 % der Theorie).

$R_F$ ($CH_2Cl_2/CH_3OH$ = 9/1) : 0,68 ;
13-C-NMR ($CDCl_3$) :
δ = 123-145 (arom. C) ; 171,11 und 170,31 (3 $\underline{\underline{C}}OCH_3$) ;
176,17 ($CO-\underset{\underset{C_2H_5}{|}}{C}H-$) ; 69,65 (C-2″) ; 99,82 ($\bar{C}$-1″) ;
96,88 (C-1′) ; 96,57 (C-4′) ; 89,14 (C-6′) ;
70,88 (C-4″) ; 55,26 (C-1) ; 47,46 (C-3).

## Beispiel 19

1,3″-Bis-N-(o-nitrophenylsulfenyl)-2′,3,6′-tris-tert.-butoxycarbonyl-2″-O-(2-ethylhexanoyl)-sisomicin

40 g rohes Penta-N-(o-nitrophenylsulfenyl)-2″-O-(2-ethylhexanoyl)-sisomicin werden wie in Beispiel 9 beschrieben zu 1,3″-Bis-N-(o-nitrophenylsulfenyl)-2″-O-(2-ethylhexanoyl)-sisomicin umgesetzt, der ausgefallene Niederschlag abgesaugt und mit 170 ml Methanol gewaschen. Das Filtrat wird mit 125 ml basischem Ionenaustauscher ($OH^{\ominus}$-Form, mit Methanol gespült) und 25 g Di-tert.-butyl-dicarbonat versetzt und 1 Stunde kräftig gerührt, filtriert und das Filtrat im Vakuum eingedampft. Man erhält 35 g (99 % der Theorie).

$R_F$ ($CH_2Cl_2/CH_3OH$ = 97,5/2,5) = 0,59 ;
IR (KBr) : 1 705 $cm^{-1}$.

## Beispiel 20

1,3″-Bis-N-(o-nitrophenylsulfenyl)-2′,3,6′-N-triacetyl-sisomicin

a) Ausgehend von 1,3″-Bis-N-(o-nitrophenylsulfenyl)-2′,3,6′-triacetyl-2″-O-[dimethyl-(1,2-dimethylpropyl)-silyl]-sisomicin.

Das nach Beispiel 10 erhaltene ungereinigte Öl wird in 100 ml Dimethylsulfoxid (DMSO) gelöst, mit 8,75 ml einer 50 proz. wäßrigen KF-Lösung versetzt und kräftig gerührt. Nach 2 Stunden wird der Ansatz auf 300 g Eis gegossen, der Niederschlag absitzen lassen und die wäßrige DMSO-Phase vorsichtig dekantiert. Der Rückstand wird mit 100 ml Wasser verrührt, die wäßrige Phase dekantiert und der Rückstand im Hochvakuum getrocknet. Man erhält 28 g (76 % bezogen auf Penta-N-(O-nitrophenylsulfenyl)-2″-O-[dimethyl-(1,2-dimethylpropyl)-silyl]-sisomicin nach Beispiel 2) rohes Produkt, welches durch Chromatographie mit $CH_2Cl_2/CH_3OH$ = 95/5 an Kieselgel weiter gereinigt werden kann.

b) Aus 1,3″-Bis-N-(o-nitrophenylsulfenyl)-2′,3,6′-N-triacetyl-2″-O-(2-ethylhexanoyl)-sisomicin.

Das rohe Öl von Beispiel 18 wird in 14 ml Dichlormethan/37,5 ml $CH_3OH$ gelöst und mit 25 ml 6 n NaOH 48 Stunden bei Raumtemperatur stehen gelassen. Nach Abdestillieren der organischen Lösungsmittel in Vakuum wird das anfallende Öl zweimal mit wenig Wasser gewaschen und getrocknet. Man erhält 3,75 g (71 %, bezogen auf Penta-N-(o-nitrophenylsulfenyl)-2″-O-(2-äthylhexanoyl)-sisomicin nach Beispiel 3).

c) Aus 1,3″-Bis-N-(o-nitrophenylsulfenyl)-2′,3,6′-N-triacetyl-2″-O-isovaleryl-sisomicin.

5,9 g der nach Beispiel 17 erhaltenen Verbindung werden nach Beispiel 20 b umgesetzt und aufgearbeitet. Man erhält 4,89 g (91 % der Theorie).

d) Aus 1,3″-Bis-N-(o-nitrophenylsulfenyl)-sisomicin.

7,52 g der nach Beispiel 21 erhaltenen Substanz werden mit Acetanhydrid und 4 n NaOH acetyliert. Das nach der Aufarbeitung anfallende Rohprodukt wird in quantitativer Ausbeute (8,7 g) isoliert.

Die nach a-d hergestellten Produkte sind in allen physikalischen Daten identisch.

$R_F$ ($CH_2Cl_2/CH_3OH$ = 9/1) : 0,37 ;

1-H-NMR (220 MHz) in $CD_3OD$ :

δ = 1,97, 1,94, 1,88 ($\underline{CH_3}CO$) ;

3,03 (3″-N-$\underline{CH_3}$) ppm ;

13-C-NMR ($CD_3OD$) :

δ = 173,0, 173,42 (drei $CH_3\underline{C}O$) ppm.

## Beispiel 21

1,3″-Bis-N-(o-nitrophenylsulfenyl)-sisomicin

a) 18,5 g 1,3″-Bis-N-(o-nitrophenylsulfenyl)-2″-O-[dimethyl-(1,3-dimethyl-propyl)-silyl]-sisomicin werden in 50 ml Dimethylsulfoxid gelöst und mit 5,4 ml 4 n NaOH versetzt. Nach 5 Minuten wird der Ansatz auf 500 ml $H_2O$ gegossen, der Niederschlag abgesaugt und im Vakuum getrocknet. Die Ausbeute beträgt 14,7 g (93 % der Theorie).

b) 17,6 g 1,3″-Bis-N-(o-nitrophenylsulfenyl)-2″-O-[dimethyl-(1,3-dimethyl-propyl)-silyl]-sisomicin in 60 ml Dimethylsulfoxid und 5 ml Wasser werden mit 20 g KF 18 Stunden kräftig gerührt und wie unter a) aufgearbeitet.

Man erhält 14 g (93 % der Theorie).

c) Das rohe Produkt von Beispiel 35 wird in 5 ml $CH_2Cl_2$ und 11 ml $CH_3OH$ gelöst und mit 2,6 ml 6 n NaOH 2 Stunden bei Raumtemperatur stehen gelassen. Das Reaktionsgemisch wird eingedampft, auf eine mit Kieselgel gefüllte Säule (7 cm lang, 5,5 cm breit) gegeben und mit 1. $CH_2Cl_2$, 2. $CH_2Cl_2/CH_3OH$ 95/5, 3. $CH_2Cl_2/CH_3OH$ 8/2 und 4. $CH_2Cl_2/CH_3OH$/20 %igem wäßrigen $NH_3$ 7/2, 7/0,3 eluiert. Man erhält 3,42 g (46 % der Theorie bezogen auf rohes Penta-N-(o-nitrophenylsulfenyl)-sisomicin.

d) Das rohe Produkt von Beispiel 36 wird analog Beispiel 21 c umgesetzt und aufgearbeitet. Man erhält 3,5 g (47 % der Theorie bezogen auf rohes Penta-N-(o-nitrophenylsulfenyl)-sisomicin.

$R_F$ (Laufmittel wie Beispiel 6) : 0,16 ;

13-C-NMR (DMSO-d6) :

δ = 124-147 (arom. C) ; 70,58 (C-2″) ;

34,66 (N−$CH_3$) ; 55,95 (C-1) ;

49,02 (C-3) ; 85,84 (C-4) ; 86,79 (C-6) ppm.

## Beispiel 22

1,3″-Bis-N-(o-nitrophenylsulfenyl)-2′,3,6′-tris-(tert.-butoxycarbonyl)-sisomicin

a) Aus 1,3″-Bis-N-(o-nitrophenylsulfenyl)-2′,3,6′-tris-N-(tert.-butoxycarbonyl-2″-O-[dimethyl-(1,2-dimethylpropyl)-silyl]-sisomicin.

Das Rohprodukt von Beispiel 11 wird in 87 ml Dimethylsulfoxid gelöst, mit 10 g gepulvertem KF sowie 5 ml $H_2O$ versetzt und kräftig gerührt. Nach 3 Stunden wird der Ansatz mit 300 ml $H_2O$ verdünnt, der Niederschlag abgesaugt, mit Wasser gewaschen und getrocknet. Die Rohausbeute ist quantitativ.

$R_F$ ($CH_2Cl_2/CH_3OH$ = 95/5) = 0,48.

b) Aus 1,3″-Bis-N-(o-nitrophenylsulfenyl)-2′,3,6′-tris-N-tert.-butoxycarbonyl-2″-O-(2-ethyl-hexanoyl)-sisomicin.

Das Rohprodukt von Beispiel 19 wird in 300 ml $CH_3OH$/60 ml $CH_2Cl_2$ gelöst und mit 60 ml 4 n NaOH 3 Tage bei Raumtemperatur stehen gelassen, das Methanol im Vakuum abgedampft, der Rückstand in Dichlormethan aufgenommen und dreimal mit Wasser ausgeschüttelt. Die organische Phase wird über $Na_2SO_4$ getrocknet und eingedampft. Die Ausbeute beträgt 18,1 g (57 % der Theorie).

## Beispiel 23

1,3″-Bis-N-(o-nitrophenylsulfenyl)-2′,3,6′-tris-N-(1,1-dimethyl-2,2,2-trichlorethyloxycarbonyl)-sisomicin

Das rohe 1,3″-Bis-N-(o-nitrophenylsulfenyl)-2′,3,6′-tris-(1,1-dimethyl-2,2,2-trichlorethyloxycarbonyl)-2″-O-[dimethyl-(1,2-dimethyl-propyl)-silyl]-sisomicin von Beispiel 12 wird in 90 ml Dimethylsulfoxid gelöst

und mit 8 ml Wasser und 5 g KF 4 Stunden gerührt. Das Reaktionsgemisch wird mit 300 ml Toluol verdünnt und das Dimethylsulfoxid mit insgesamt 500 ml Wasser ausgeschüttekt. Nach dem Trocknen und Eindampfen der organischen Phase wird der gelbe Rückstand ohne Reinigung weiterverarbeitet.

$R_F$ ($CH_2Cl_2/CH_3OH$ = 97,5/2,5) = 0,42.

Beispiel 24

1,3"-Bis-N-(o-nitrophenylsulfenyl)-2',3,6'-tris-N-(4-methoxybenzyloxycarbonyl)-sisomicin

5 g Rohprodukt von Beispiel 15 werden in 12 ml Dimethylsulfoxid und 1 ml Wasser gelöst und mit 0,9 g KF 4 Stunden bei Raumtemperatur gerührt. Der Ansatz wird mit 50 ml Dichlormethan verdünnt und das Dimethylsulfoxid mit je 10 ml Wasser 3 mal ausgeschüttelt. Das nach Eindampfen der organischen Phase zurückbleibende Rohprodukt wird ohne weitere Reinigung weiterverarbeitet.

$R_F$ ($CH_2Cl_2/CH_3OH$ = 95/5) = 0,52.

Beispiel 25

1,3"-Bis-N-(o-nitrophenylsulfenyl-2',3,6'-tris-N-(2,2,2-trichlorethyloxycarbonyl)-sisomicin

11 g des Rohprodukts von Beispiel 13 werden in 24 ml Dimethylsulfoxid und 1,3 ml Wasser gelöst und mit 0,6 g KF 1 Stunde gerührt. Das Produkt wird mit $H_2O$ ausgefällt und im Vakuum getrocknet. Die Ausbeute beträgt 98 % der Theorie.

$R_F$ ($CH_2Cl_2/CH_3OH$ = 97,5/2,5) = 0,32.

Beispiel 26

1,3"-Bis-N-(o-nitrophenylsulfenyl)-2',3,6'-tris-N-trichloracetyl-sisomicin

Das rohe Öl von Beispiel 14 wird in 20 ml Dimethylsulfoxid gelöst, mit 2 ml einer 50 proz. KF-Lösung versetzt und 3 Stunden kräftig gerührt. Das Produkt wird mit Wasser ausgefällt, mit Wasser gewaschen und getrocknet. Das Rohprodukt wird ohne weitere Reinigung weiterverarbeitet.

$R_F$ ($CH_2Cl_2/CH_3OH$ = 97,5/2,5) = 0,42 ;
13-C-NMR ($CDCl_3$) :
δ = 103,60 (C-1") ; 66,48 (C-3") ; 55,15 (C-1) ;
50,60 (C-3) ; 79,86 (C-4) ; 76,18 (C-5) ;
89,16 (C-6) ; 97,74 (C-1') ; 96,84 (C-4') ;
149,80 (C-5) ; 92,78 ($CCl_3$) ; 162,29 und
162,11 (CO) ppm.

Beispiel 27

3"-N-(o-nitrophenylsulfenyl)-2',3,6'-tris-N-acetyl-sisomicin

28 g Rohprodukt von Beispiel 20 werden in 56 ml Dichlormethan gelöst, mit 18 g 2-Mercaptobenzthiazol sowie 93 ml Methanol versetzt und kräftig geschüttelt, bis eine klare Lösung entstanden ist. Der Ansatz wird 20 Stunden bei 5 °C gehalten und tropfenweise mit 16 ml 12 proz. $H_2O_2$-Lösung versetzt. Der Niederschlag wird abgesaugt und das Filtrat in Vakuum eingedampft. Man erhält einen roten Schaum, der für die meisten Folgeumsetzungen ohne weitere Reinigung eingesetzt werden kann. Die Ausbeute beträgt 26,8 g (88,6 %, bezogen auf rohes Penta-N-(o-nitrophenylsulfenyl)-2"-O-[dimethyl-(1,2-dimethylpropyl)-silyl]-sisomicin nach Beispiel 2). Der Schaum besteht zu 57 % aus dem gewünschten produkt, d.h. die Gesamtausbeute, bezogen auf Beispiel 2 beträgt 51 % der Theorie. Zur weiteren Reinigung werden 3 g des Rohprodukts an Kieselgel [Säule 3,8 × 30 cm, Laufmittel $CH_2Cl_2/CH_3OH$ = 9/1 mit steigendem Zusatz (zuletzt 5 %) einer Mischung aus $CH_2Cl_2/CH_3OH$/20 % wäßriges $NH_3$ = 2/4/1] chromatographiert. Die Ausbeute ist 1,4 g.

$R_F$ ($CH_2Cl_2/CH_3OH$/20 % wäßriges $NH_3$ = 7,5:2,4:0,15) : 0,79 ;
13-C-NMR (d6-DMSO) :
71,70 (C-2") ; 50,89 (C-1) ; 47,73 (C-3) ; 80,95 (C-4) ;
74,79 (C-5) ; 86-53 (C-6) ; 45,52 (C-2') ; 96,55 (C-1') ;
146,78 (C-5') ; 169,23, 168,94, 169,64 (3 × CO) ppm.

Beispiel 28

3"-N-(o-nitrophenylsulfenyl)-2',3,6'-tris-N-tert.-butoxycarbonyl-sisomicin

Das Rohprodukt von Beispiel 22 a) wird in 89 ml Dichlormethan gelöst, mit 30 g 2-Mercaptobenzthiazol sowie 250 ml Methanol kräftig bis zur klaren Lösung geschüttelt und 28 Stunden bei 5 °C stehen gelassen. Nach Absaugen des Niederschlags wird das Filtrat rasch im Vakuum eingedampft, der Rückstand mit Toluol aufgenommen und bis zur völligen Entfernung des 2-Mercaptobenzthiazols mit 4 n NaOH extrahiert. Nach Trocknen der organischen Phase über $Na_2SO_4$ wird das Lösungsmittel im Vakuum abgedampft. Der Rückstand (25,1 g = 67 % über alle Stufen, ausgehend von Beispiel 2) ist für weitere Umsetzungen genügend rein.

$R_F$ ($CH_2Cl_2/CH_3OH$/20 % wäßriges $NH_3$ 93/6, 5/0,5) = 0,49.

Beispiel 29

3″-N-(o-nitrophenylsulfenyl)-2′,3,6′-tris-N-(1,1-dimethyl-2,2,2-trichlor-ethyloxycarbonyl)-sisomicin

Ausgehend von dem Rohprodukt aus Beispiel 23 erfolgt die Umsetzung analog Beispiel 28. Der erhaltene Abdampfrückstand liefert nach der Chromatographie an Kieselgel (Laufmittel $CH_2Cl_2/CH_3OH$ = 95/5) 21,7 g (43 % bezogen auf Beispiel 2) reines Endprodukt.

$R_F$ ($CH_2Cl_2/CH_3OH$ = 95/5) : 0,22 ;
($CH_2Cl_2/CH_3OH$ = 90/10) : 0,77 ;
13-C-NMR ($CDCl_3$) :
δ = 70,68 (C-2″) ; 38,41 (N–$\underline{C}H_3$) ; 50,87 (C-1) ;
50,53 (C-3) ; 79,81 (C-4) ; 89,94 (C-6) ;
98,24 (C-1′) ; 47,45 (C-2′) ; 97,52 (C-4′) ;
146,35 (C-5′) ; 42,35 (C-6′) ppm.

Beispiel 30

3″-N-(o-nitrophenylsulfenyl)-2′,3,6′-tris-N-(p-methoxybenzyloxycarbonyl)-sisomicin

Das Rohprodukt von Beispiel 24 wird in 19 ml Dichlormethan gelöst und mit 20 ml Methanol sowie 4,9 g 2-Mercaptobenzthiazol geschüttelt bis zur klaren Lösung. Nach 3 Tagen bei 5 °C wird der Niederschlag abfiltriert, das Filtrat eingedampft und an Kieselgel (Säule 3,5 × 11 cm, Laufmittel anfangs $CH_2Cl_2/CH_3OH$ = 97,5/2,5, zuletzt $CH_2Cl_2/CH_3OH$ = 95/5) chromatographiert. Die Ausbeute an reinem Produkt beträgt 2,1 g (52 %, bezogen auf Beispiel 15).

$R_F$ ($CH_2Cl_2/CH_3OH$/20 % wäßriges $NH_3$ 93/6, 5/0,5) = 0,49.

Beispiel 31

3″-N-(o-nitrophenylsulfenyl)-2′,3,6′-tris-N-trichloracetyl-sisomicin

Das produkt von Beispiel 26 wird in 13 ml Dichlormethan gelöst, mit 26 ml Methanol und 5 g 2-Mercaptobenzthiazol bis zur klaren Lösung geschüttelt und 3 Tage bei 5 °C stehen gelassen. Der Niederschlag wird abfiltriert, das Filtrat eingedampft und an Kieselgel chromatographiert (Laufmittel a : $CH_2Cl_2/CH_3OH$ = 95/5 ; b : ($CH_2Cl_2/CH_3OH$/20 % wäßriges $NH_3$ = 93/6, 5/0,5).

$R_F$ : ($CH_2Cl_2/CH_3OH$/20 % wäßriges $NH_3$ = 93/6, 5/0,5) : 0,43 ;
13-C-NMR ($CDCl_3$) :
δ = 103,43 (C-1″) ; 67,46 (C-3″) ; 50,85 (C-1) ;
50,28 (C-3) ; 79,44 (C-4) ; 76,51 (C-5) ;
89,29 (C-6) ; 97,61 (C-1′) ; 96,62 (C-4′) ;
149,50 (C-5′) ; 92,46 und 92,38 (C-4′) ;
162,01 und 161,76 (CO) ppm.

Beispiel 32

3″-N-(o-nitrophenylsulfenyl)-2′,3,6′-tris-N-(2,2,2-trichloräthyloxycarbonyl)-sisomicin

9,8 g Rohprodukt von Beispiel 25 werden in 9 ml Dichlormethan gelöst und mit 16 ml Methanol und 1,45 g 2-Mercaptobenzthiazol bis zur klaren Lösung geschüttelt und dann bei Raumtemperatur 6 Tage stehen gelassen. Der Niederschlag wird abfiltriert und das Filtrat an Kieselgel (Laufmittel wie in Beispiel 31) chromatographiert.

$R_F$ (Laufmittel b von Beispiel 31) : 0,53.

Beispiel 33

Penta-N-(o-nitrophenylsulfenyl)-2″-O-(2,2-dimethylhexyloxycarbonyl)-sisomicin

12 g rohes Penta-N-(o-nitrophenylsulfenyl)-sisomicin in 50 ml absolutem Dichlormethan werden mit

4,3 ml Triäthylamin und 1 g p-Dimethylaminopyridin sowie bei 0 °C mit 5,8 ml 2,2-Dimethylhexyloxycarbonylchlorid versetzt und bei Raumtemperatur 40 Stunden gerührt. Das Reaktionsgemisch wird 2 mal mit je 15 ml gesättigter $NaHCO_3$-Lösung gewaschen, über Natriumsulfat getrocknet und mit Petroläther zur Ausfällung des gewüngschten Produktes versetzt. Die Ausbeute nach dem Trocknen ist quantitativ.

$R_F$ ($CH_2Cl_2/CH_3OH$ 99/1) = 0,57.

### Beispiel 34

Penta-N-(o-nitrophenylsulfenyl)-2"-O-(2,2-dimethylpropyloxycarbonyl)-sisomicin

12 g rohes Penta-N-(o-nitrophenylsulfenyl)-sisomicin werden mit 6 ml 2,2-Dimethylpropyloxycarbonylchlorid analog Beispiel 33 umgesetzt und aufgearbeitet. Das Produkt wird in quantitativer Ausbeute isoliert.

$R_F$ ($CH_2Cl_2/CH_3$ 99/1) = 0,53.

### Beispiel 35

1,3"-Bis-N-(o-nitrophenylsulfenyl)-2"-O-(2,2-dimethylhexyloxycarbonyl)-sisomicin

Das Rohprodukt von Beispiel 33 wird mit 18 ml Dichlormethan, 26 ml Methanol und 3,7 g 2-Mercaptobenzthiazol bis zur klaren Lösung geschüttelt und 70 Stunden bei Raumtemperatur stehengelassen. Ser nach dem Abfiltrieren des entstandenen Niederschlages und Eindampfen des Filtrates erhaltene Festkörper hat einen $R_F$-Wert ($CH_2Cl_2/CH_3OH$/20 %iges wäßriges $NH_3$ 7/2, 7/0,3) von 0,61 und wird ohne weitere Reinigung entsprechend Beispiel 21c, weiterverarbeitet.

### Beispiel 36

1,3"-Bis-N-(o-nitrophenylsulfenyl)-2"-O-(2,2-dimethylpropyloxycarbonyl)-sisomicin

Aus dem Rohprodukt von Beispiel 34 erhält man analog zu Beispiel 35 einen Feststoff mit dem $R_F$-Wert ($CH_2Cl_2/CH_3OH$/20 %iges wäßriges $NH_3$ 7/2, 7/0,3) von 0,62, der nach Beispiel 21d, weiterverarbeitet wird.

### Beispiel 37

1-(2-Aminoethyloxycarbonyl)-sisomicin

a) (4-Nitrophenyl)-[2-(o-nitrophenylsulfenylamino)-ethyl]-carbonat

Man legt eine Lösung von 1,4 g 2-Aminoäthanol in 30 ml Dioxan vor und tropft gleichzeitig unter Aufrechterhaltung von pH 8 eine Lösung von 3,8 g o-Nitrophenylsulfensäurechlorid in 10 ml Dioxan und 8,5 ml 2n-Natronlauge zu. Nach mehrstündigem Rühren bei Raumtemperatur wird im Vakuum eingeengt, der Rückstand in Essigester aufgenommen und zweimal mit Wasser gewaschen, mit $Na_2SO_4$ getrocknet und im Vakuum eingeengt. Das zurückbleibende Öl wird mit Toluol/Essigester (2 : 1) über 100 g Kieselgel chromatographiert und die Hauptkomponente abgetrennt. Ausbeute : 2,9 g N-(2-Hydroxyäthyl)-o-nitrosulfensäureamid.

456 mg dieser Verbindung und 600 mg Chlorameisensäure-p-nitrophenylester werden in 5 ml Acetonitril gelöst und unter Eiskühlung mit 300 mg Triäthylamin in 5 ml Acetonitril versetzt. Nach einer Stunde bei Raumtemperatur wird im Vakuum eingeengt, in 30 ml Methylenchlorid aufgenommen, zweimal mit Wasser gewaschen, mit $Na_2SO_4$ getrocknet, im Vakuum eingeengt und das erhaltene Öl mit Toluol/Essigester (2 : 1) über 100 g Kieselgel chromatographiert und die Hauptfraktion abgetrennt. Ausbeute : 250 ml oranges Öl, das langsam durchkristallisiert.

IR(Kpr) : 1 770 $cm^{-1}$ ; $R_F$-Wert Toluol/Essigester (2 : 1) : 0,77.

b) 5 g 3"-N-(o-nitrophenylsulfenyl)-2',3,6'-tris-N-trichloracetylsisomicin werden in 10 ml Pyridin gelöst und mit 2,2 g rohem Produkt von Beispiel 37a) versetzt.

Nach 2 Stunden wird der Ansatz im Vakuum eingedampft, in 30 ml Dichlormethan/30 ml Methanol aufgenommen und mit 10 ml 4n NaOH versetzt. Nach 4 Stunden wird wiederum im Vakuum eingedampft, mit 1,7 g 2-Mercaptobenzthiazol in 10 ml Dichlormethan/3 ml Methanol versetzt und mit halbkonzentriertem methanolischem HCl vorsichtig angesäuert. Nach Zugabe von 10 ml Wasser wird der Ansatz mit basischem Ionenaustauscher ($OH^{\ominus}$-Form) auf pH = 10 gebracht, die wäßrige Phase abgetrennt, eingedampft und an Kieselgel chromatographiert (Laufmittel : $CH_2Cl_2/CH_3OH$/20 % wäßriges $NH_3$ = 2/4/1). Die Ausbeute beträgt 1 930 mg (75 % der Theorie).

$R_F$ = 0,28 ($CH_2Cl_2/CH_3OH$/20 % konzentriertes $NH_3$ 2/4/1).

Beispiel 38

1-N-[(S,R,R)-2,3,4,5-Tetrahydroxypentyl]-sisomicin

1 g 2',3,6'-Tri-N-acetyl-3''-N-(o-nitrophenylsulfenyl)-sisomicin wird in 33 ml Methanol und 6,6 ml Wasser gelöst. Man fügt 1 g D-Xylose hinzu und erhitzt 30 Minuten auf 70 °C. Nach Zugabe von 330 mg Natriumcyanoborhydrid erhitzt man eine weitere Stunde, läßt abkühlen und entionisiert durch Rühren mit basischem Ionenaustauscherharz ($OH^{\ominus}$-Form). Man dampft im Vakuum zu einem amorphen Feststoff ein.

Zur Abspaltung der Schutzgruppen erhitzt man in 6 ml gesättigter Bariumhydroxidlösung 5 Stunden am Rückfluß. Man entfernt die Bariumionen durch Zugabe von Schwefelsäure bis pH 5 und anschließendes Zentrifugieren. Das Zentrifugat wird durch Ausrühen mit basischem Ionenaustauscherharz ($OH^{\ominus}$-Form) entionisiert, mit 150 ml Methylenchlorid gewaschen und anschließend mit Holzkohle ausgerührt. Man filtriert und dampft im Vakuum zum farblosen Feststoff ein.

$R_F$-Wert = 0,3 (Laufmittelsystem $CH_2Cl_2/CH_3OH$/konz. $NH_3$ = 2/2/1).

Beispiel 39

1-N-Ethyl-sisomicin

364 mg 2',3,6'-Tri-N-acetyl-3''-N-(o-nitrophenylsulfenyl)-sisomicin in 3 ml Wasser werden mit einer Lösung von 25 µl Acetaldehyd in 1,5 ml Methanol 30 Minuten bei Raumtemperatur gerührt. Man fügt dann 100 mg Natriumcyanoborhydrid hinzu und versetzt nach einer weiteren Stunde mit 3 µl Acetaldehyd in 0,2 ml Methanol. Nach einer Stunde wird im Vakuum eingedampft, der Rückstand in 10 ml Wasser mit basischem Ionenaustauscherharz ($OH^{\ominus}$-Form) behandelt und nach Abfiltrieren vom Harz im Vakuum zur Trockne eingedampft. Zur Abspaltung der Schutzgruppen erhitzt man die Substanz in 2 ml gesättigter Bariumhydroxidlösung 5 Stunden zur Rückfluß. Man arbeitet wie in Beispiel 34 auf und erhält 1-N-Ethylsisomicin als farblosen, amorphen Feststoff.

**Ansprüche** (für die Vertraagsstaaten : BE, CH, DE, FR, GB, IT, NL, SE)

**1.** Verbindungen der allgemeinen Formel

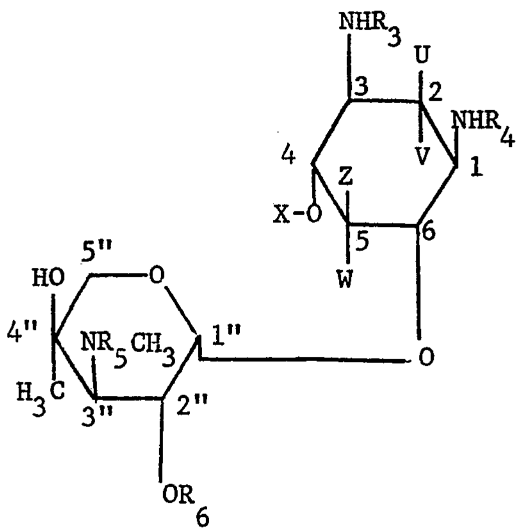

in der

X für einen Rest der Formeln

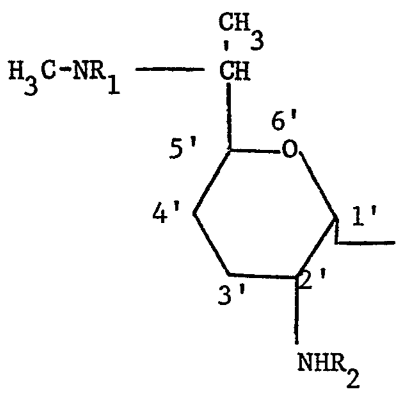

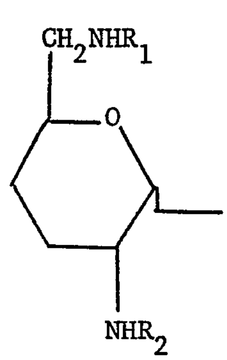

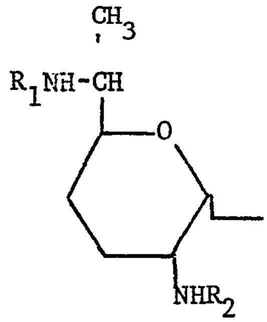

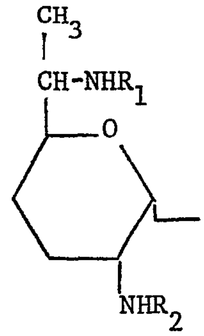

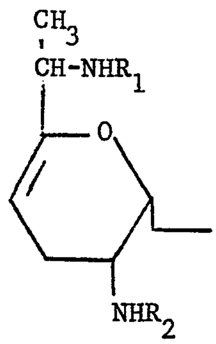

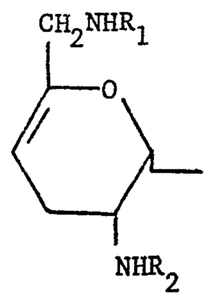

U, V, W und Z für Wasserstoff oder Hydroxy,
$R_1$, $R_2$ und $R_3$ für $-CO-A$,
$R_4$ für Wasserstoff,
$R_5$ für einen Rest $-SR_7$ und
$R_6$ für Wasserstoff oder einen abspaltbaren, raumerfüllenden Substituenten stehen,
wobei U und V sowie W und Z nicht gleichzeitig OH und wobei A einen Rest der Formeln

$-CHal_3$, $-(CH_2)_nB$, $-O-E$, 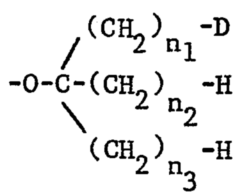 oder 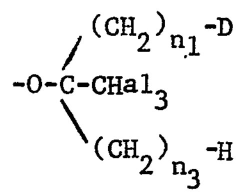

B und D Wasserstoff oder gegebenenfalls substituiertes Phenyl,
E gegebenenfalls substituiertes Phenyl,
n eine Zahl von 0-5,
$n_1$, $n_2$ und $n_3$ eine Zahl von 0-5,
Hal Fluor, Chlor oder Brom und
$R_7$ gegebenenfalls substituiertes Phenyl, Di- oder Triphenylmethyl
bedeuten.

**2.** Verbindungen gemäß Anspruch 1, wobei $R_6$ für Wasserstoff oder einen Rest der Formeln

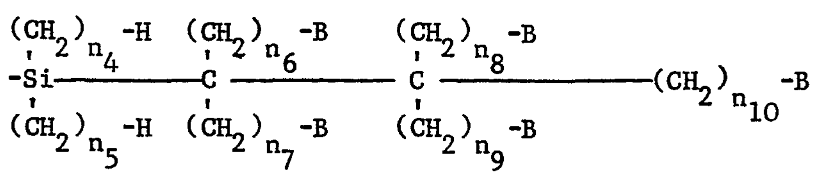 oder

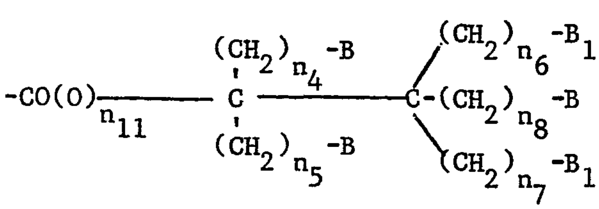 steht und

worin

$n_4$, $n_5$, $n_6$, $n_7$, $n_8$, $n_9$, $n_{10}$ eine Zahl 0-3,

$n_{11}$ eine Zahl 0 oder 1,

$B_1$ B, $C_1$-$C_5$-Alkoxy oder $C_3$-$C_5$-Alkenyloxy bedeuten und

B, D und E Wasserstoff oder gegebenenfalls 1 oder 2 mal durch Nitro, Halogen, $C_1$-$C_4$-Alkyl, $C_1$-$C_4$-Alkoxy oder Phenyl substituiertes Phenyl und

$R_7$ genebenenfalls ein- bis dreimal durch $CF_3$, $NO_2$, $C_1$-$C_4$-Alkyl, $C_1$-$C_4$-Alkoxy, $C_1$-$C_4$-Alkoxycarbonyl oder Phenyl oder ein- bis fünfmal durch Halogen substituiertes Phenyl Diphenylmethyl oder Triphenylmethyl bedeuten.

**3.** Verbindungen gemäß Anspruch 1 der allgemeinen Formel

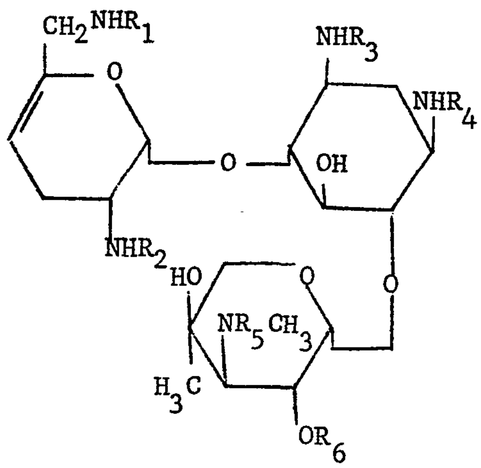

worin

$R_1$, $R_2$, $R_3$, $R_4$, $R_5$ und $R_6$ die in Anspruch 1 angegebene Bedeutung haben.

**4.** Verfahren zur Herstellung von Verbindungen gemäß Anspruch 1, dadurch gekennzeichnet, daß man Verbindungen der Formel

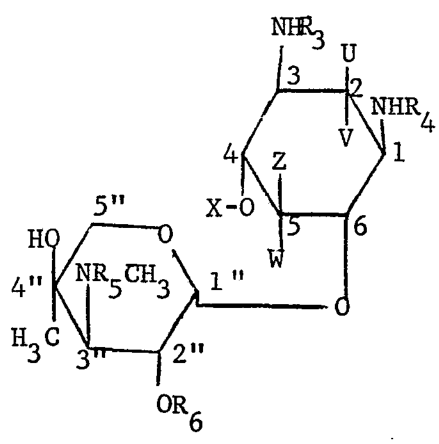

worin

$R_1$, $R_2$, $R_3$, $R_4$ und $R_5$ eine Gruppe $-SR_7$, und

$R_6$ Wasserstoff bedeuten und

U, V, W, Z, X und $R_7$ die in Anspruch 1 bekannte Bedeutung haben,

1. mit einem Silylierungsmittel oder einem Acylierungsmittel der Formel

$$R_6-G$$

worin

$R_6$ für einen abspaltbaren, raumerfüllenden Substituenten und
G für eine Abgangsgruppe steht
umsetzt,

2. die $-S-R_7$-Gruppen $R_1$, $R_2$, $R_3$ und gegebenenfalls $R_4$ abspaltet,

3. die freigesetzten Aminogruppen mit Ausnahme der 1-$NH_2$-Gruppe, falls diese im Schritt 2. bereits freigesetzt wurde mit Acylierungsmitteln der Formel

$$A-CO-G \qquad (IV)$$

worin
A die in Anspruch 1 genannte Bedeutung hat und
G eine Abgangsgruppe bedeutet,
acyliert,

4. Falls nicht unter 2. geschehen, die $S-R_7$-Gruppe $R_4$ abspaltet und genebenenfalls

5. den raumerfüllenden Substituenten $R_6$ abspaltet, wobei die Schritte 4. und 5. in ihrer Reihenfolge auch vertauscht werden können.

**5.** Verwendung der Verbindungen gemäß Anspruch 1 zur Herstellung von 4,6-Di-O-(aminoglykosyl)-1,3-diaminocyclitolen.

**Ansprüche** (für den Vertragsstaat : AT)

**1.** Verfahren zur Herstellung von Verbindungen der allgemeinen Formel

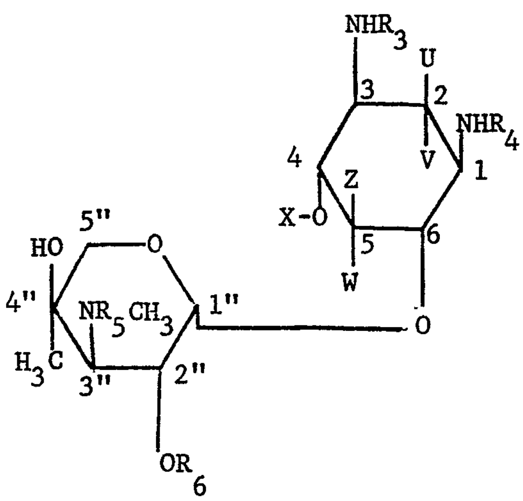

in der
X für einen Rest der Formeln

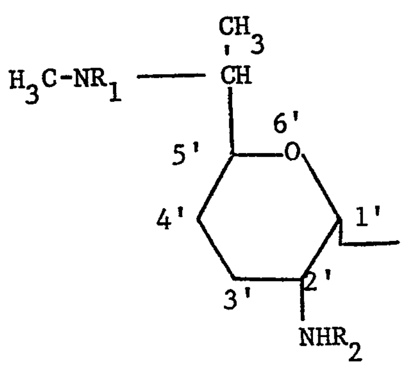

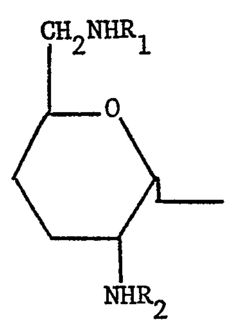

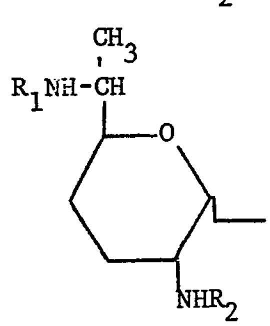

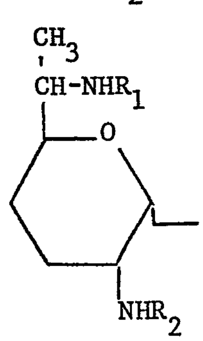

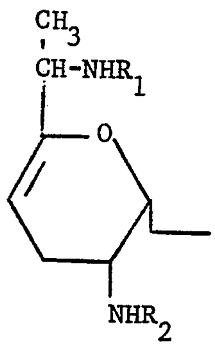
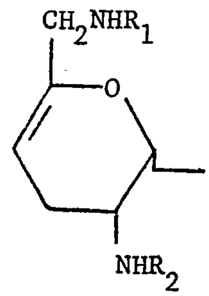

U, V, W und Z für Wasserstoff oder Hydroxy,
$R_1$, $R_2$ und $R_3$ für $-CO-A$,
$R_4$ für Wasserstoff
$R_5$ für einen Rest $-SR_7$ und
$R_6$ für Wasserstoff oder einen abspaltbaren, raumerfüllenden Substituenten stehen,
wobei U und V sowie W und Z nicht gleichzeitig OH und wobei A einen Rest der Formeln

$-CHal_3$, $-(CH_2)_nB$, $-O-E$, 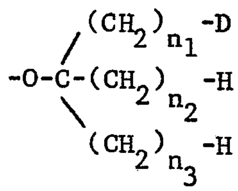 oder 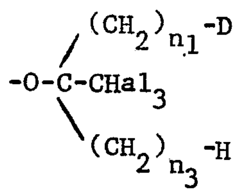

B und D Wasserstoff oder gegebenenfalls substituiertes Phenyl,
E gegebenenfalls substituiertes Phenyl,
n eine Zahl von 0-5,
$n_1$, $n_2$ und $n_3$ eine Zahl von 0-5,
Hal Fluor, Chlor oder Brom und
$R_7$ gegebenenfalls substituiertes Phenyl, Di- oder Triphenylmethyl
bedeuten, dadurch gekennzeichnet, daß man Verbindungen der Formel

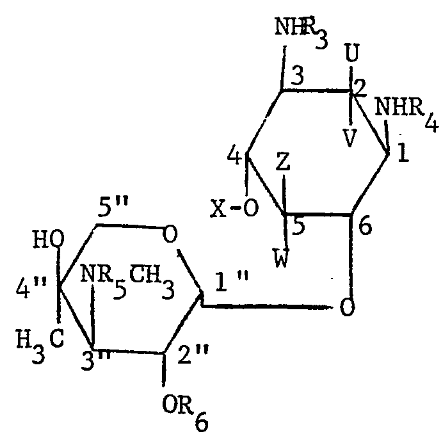

worin
X, U, V, W und Z die oben angegebene Bedeutung haben,
$R_1$, $R_2$, $R_3$, $R_4$ und $R_5$ eine Gruppe $-SR_7$ und
$R_6$ Wasserstoff bedeuten
1. mit einem Silylierungsmittel oder einem Acylierungsmittel der Formel

$$R_6-G$$

worin
$R_6$ für einen abspaltbaren, raumerfüllenden Substituenten und

G für eine Abgangsgruppe steht

umsetzt,

2. die $-S-R$-Gruppen $R_1$, $R_2$, $R_3$ und gegebenenfalls $R_4$ abspaltet,

3. die freigesetzten Aminogruppen mit Ausnahme der $1-NH_2$-Gruppe, falls diese im Schritt 2. bereits freigesetzt wurde mit Acylierungsmitteln der Formel

$$A-CO-G \qquad (IV)$$

worin

A die oben angegebene Bedeutung hat und

G eine Abgangsgruppe bedeutet,

acyliert,

4. Falls nicht unter 2. geschehen, die $S-R_7$-Gruppe $R_4$ abspaltet und gegebenenfalls

5. den raumerfüllenden Substituenten $R_6$ abspaltet, wobei die Schritte 4. und 5. in ihrer Reihenfolge auch vertauscht werden können.

**2.** Verfahren nach Anspruch 1, dadurch gekennzeichnet, daß $R_6$ für Wasserstoff oder einen Rest der Formeln

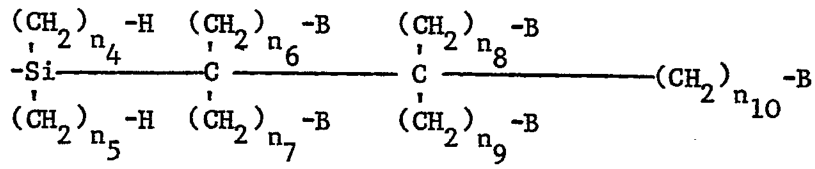

oder

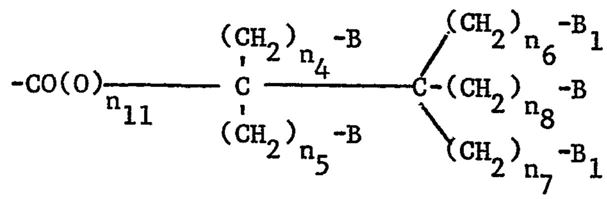

steht und

worin

$n_4$, $n_5$, $n_6$, $n_7$, $n_8$, $n_9$, $n_{10}$ eine Zahl 0-3,

$n_{11}$ eine Zahl 0 oder 1,

$B_1$ B, $C_1$-$C_5$-Alkoxy oder $C_3$-$C_5$-Alkenyloxy bedeuten und

B, D und E Wasserstoff oder gegebenenfalls 1 oder 2 mal durch Nitro, Halogen, $C_1$-$C_4$-Alkyl, $C_1$-$C_4$-Alkoxy oder Phenyl substituiertes Phenyl und

$R_7$ gegebenenfalls ein- bis dreimal durch $CF_3$, $NO_2$, $C_1$-$C_4$-Alkyl, $C_1$-$C_4$-Alkoxy, $C_1$-$C_4$-Alkoxycarbonyl oder Phenyl oder ein- bis fünfmal durch Halogen substituiertes Phenyl Diphenylmethyl oder Triphenylmethyl bedeuten.

**3.** Verfahren nach Anspruch 1 oder 2, dadurch gekennzeichnet, daß man von Verbindungen der allgemeinen Formel

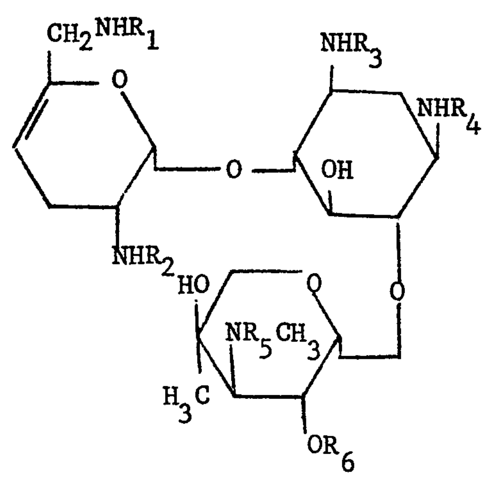

worin

$R_1$, $R_2$, $R_3$, $R_4$, $R_5$ und $R_6$ die in Anspruch 1 angegebene Bedeutung haben, ausgeht.

**4.** Verwendung der gemäß Anspruch 1 bis 3 hergestellten Verbindungen zur Herstellung von 4,6-Di-O-(Aminoglycosyl)-1,3-diaminocyclitolen.

**Claims** (for the contracting States : BE, CH, DE, FR, GB, IT, NL and SE)

**1.** Compounds of the general formula

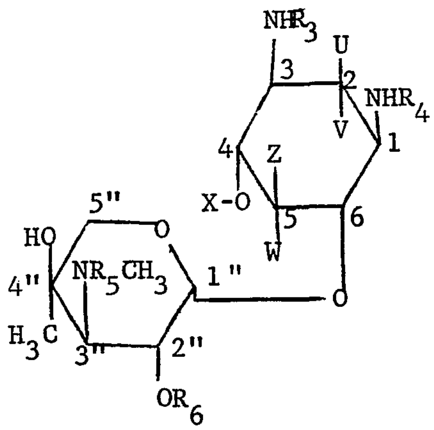

in which

X represents a radical of the formulae

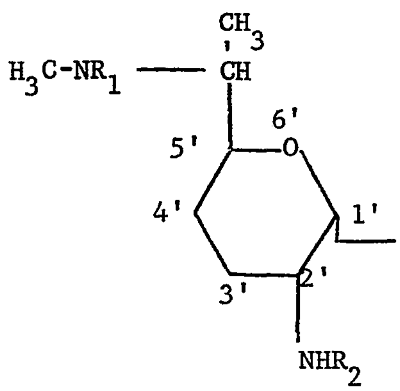

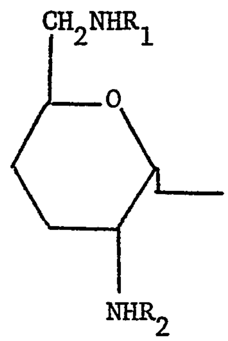

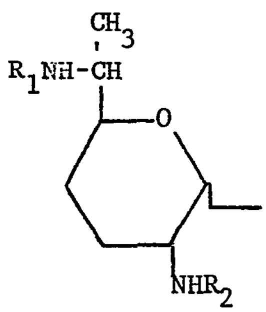

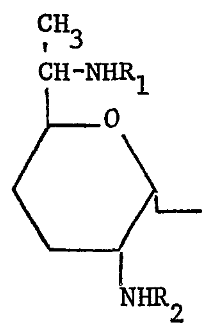

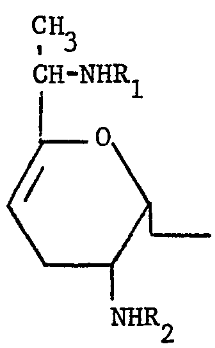

or

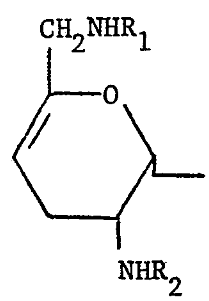

U, V, W and Z represent hydrogen or hydroxyl,
$R_1$, $R_2$ and $R_3$ represent $-CO-A$,
$R_4$ represents hydrogen,
$R_5$ represents a radical $-SR_7$ and
$R_6$ represents hydrogen or a space-filling substituent which can be split off,
U and V, and W and Z not simultaneously denoting OH, and
wherein
A denotes a radical of the formulae

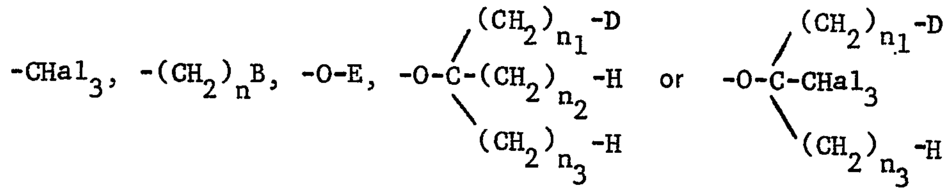

B and D denote hydrogen or optionally substituted phenyl,
E denotes optionally substituted phenyl,
n denotes a number from 0 to 5,
$n_1$, $n_2$ and $n_3$ denote a number from 0 to 5,
Hal denotes fluorine, chlorine or bromine and
$R_7$ denotes optionally substituted phenyl, diphenylmethyl or triphenylmethyl.

**2.** Compounds according to Claim 1
wherein
$R_6$ represents hydrogen or a radical of the formulae

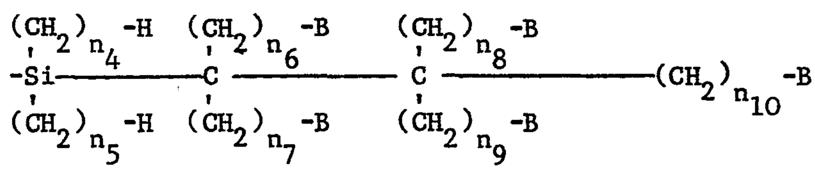 or

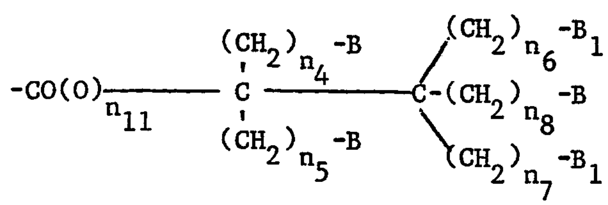

and where in
$n_4$, $n_5$, $n_6$, $n_7$, $n_8$, $n_9$ and $n_{10}$ denote a number from 0 to 3,
$n_{11}$ denotes the number 0 or 1,
$B_1$ denotes B, $C_1$-$C_5$-alkoxy or $C_3$-$C_5$-alkenyloxy and
B, D and E denote hydrogen or phenyl which is optionally monosubstituted or disubstituted by nitro, halogen, $C_1$-$C_4$-alkyl, $C_1$-$C_4$-alkoxy or phenyl and
$R_7$ denotes phenyl diphenylmethyl or triphenylmethyl which is optionally monosubstituted to trisubstituted by $CF_3$, $NO_2$, $C_1$-$C_4$-alkyl, $C_1$-$C_4$-alkoxy, $C_1$-$C_4$-alkoxycarbonyl or phenyl or monosubstituted to pentasubstituted by halogen.

**3.** Compounds according to Claim 1, of the general formula

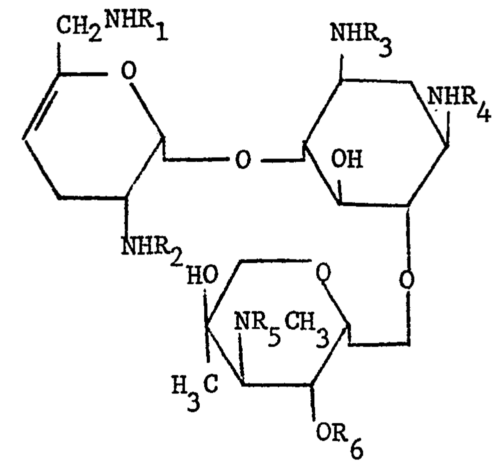

wherein

$R_1$, $R_2$, $R_3$, $R_4$, $R_5$ and $R_6$ have the meaning indicated in Claim 1.

**4.** Process for the preparation of compounds according to Claim 1, characterised in that compounds of the formula

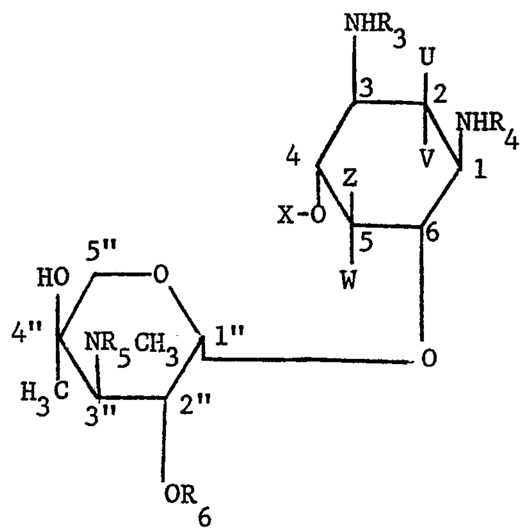

wherein

$R_1$, $R_2$, $R_3$, $R_4$ and $R_5$ denote a $-SR_7$ group,

$R_6$ denotes hydrogen and

U, V, W, Z and $R_7$ have the meaning known in Claim 1,

1. are reacted with a silylating agent or an acylating agent of the formula

$$R_6-G$$

wherein

$R_6$ represents a space-filling substituent which can be split off and

G represents a leaving group,

2. the $-S-R_7$ groups $R_1$, $R_2$, $R_3$ and, if appropriate, $R_4$ are split off,

3. the amino groups liberated, with the exception of the $1-NH_2$ group if this has already been liberated in step 2, are acylated with acylating agents of the formula

$$A-CO-G \qquad (IV)$$

wherein

A has the meaning given in Claim 1 and

G denotes a leaving group,

4. The $S-R_7$ group $R_4$ is split off, if this has not been effected under 2., and, if appropriate,

5. the space-filling substituent $R_6$ is split off, it being possible to change the sequence of steps 4. and 5.

5. Use of the compounds according to Claim 1 for the preparation of 4,6-di-O-(aminoglycosyl)-1,3-diaminocyclitols.

**Claims** (for the contracting state : AT)

1. Process for the preparation of compounds of the general formula

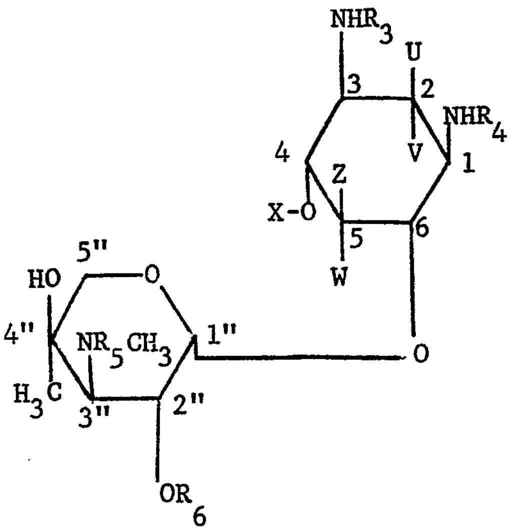

in which

X represents a radical of the formulae

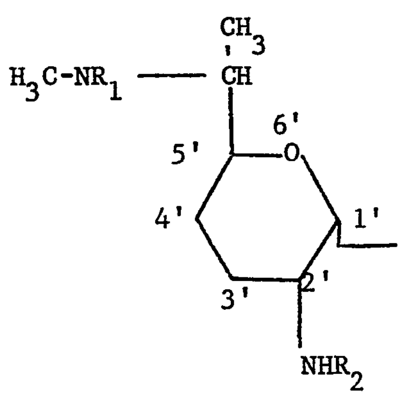

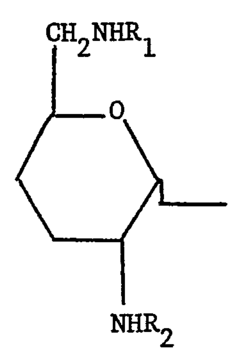

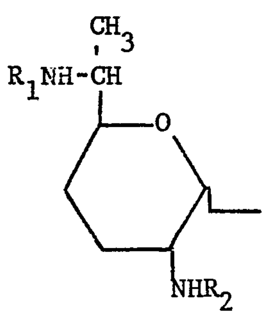

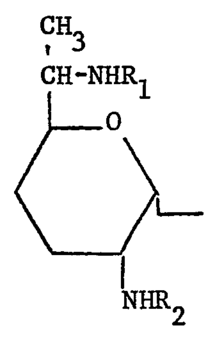

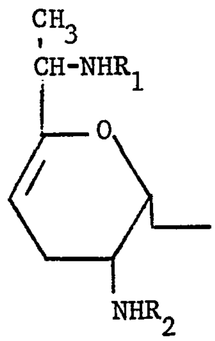

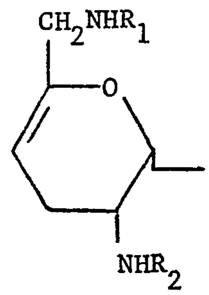

U, V, W and Z represent hydrogen or hydroxyl,
$R_1$, $R_2$ and $R_3$ represent $-CO-A$,
$R_4$ represents hydrogen,
$R_5$ represents a radical $-SR_7$ and
$R_6$ represents hydrogen or a space-filling substituent which can be split off,
U and V, and W and Z not simultaneously denoting OH, and
wherein
A denotes a radical of the formulae

$-CHal_3$, $-(CH_2)_nB$, $-O-E$, 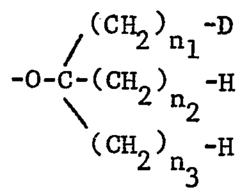 or 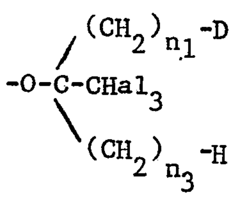

B and D denote hydrogen or optionally substituted phenyl,
E denotes optionally substituted phenyl,
n denotes a number from 0 to 5,
$n_1$, $n_2$ and $n_3$ denote a number from 0 to 5,
Hal denotes fluorine, chlorine or bromine and
$R_7$ denotes optionally substituted phenyl, diphenylmethyl or triphenylmethyl,
characterised in that compounds of the formula

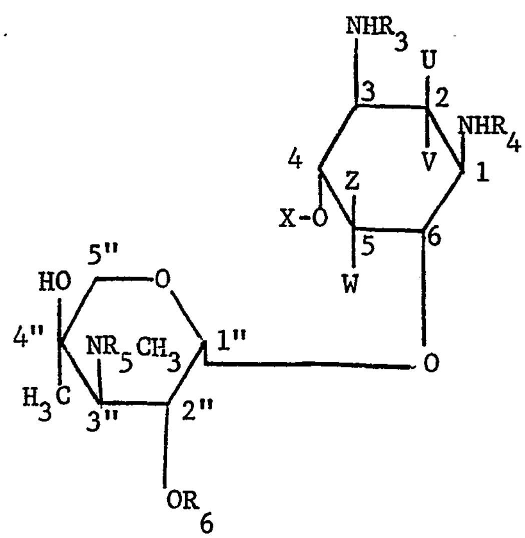

wherein
$R_1$, $R_2$, $R_3$, $R_4$ and $R_5$ denote a $-SR_7$ group,
$R_6$ denotes hydrogen
1. are reacted with a silylating agent or an acylating agent of the formula

$$R_6-G$$

wherein
$R_6$ represents a space-filling substituent which can be split off and
G represents a leaving group,

2. the $-S-R_7$ groups $R_1$, $R_2$, $R_3$ and, if appropriate, $R_4$ are split off,

3. the amino groups liberated, with the exception of the $1-NH_2$ group if this has already been liberated in step 2, are acylated with acylating agents of the formula

$$A-CO-G \qquad (IV)$$

wherein

G denotes a leaving group,

4. The $S-R_7$ group $R_4$ is split off, if this has not been effected under 2., and, if appropriate,

5. the space-filling substituent $R_6$ is split off, it being possible to change the sequence of steps 4. and 5.

**2.** Process according to Claim 1, characterised in that $R_6$ represents hydrogen or a radical of the formulae

, 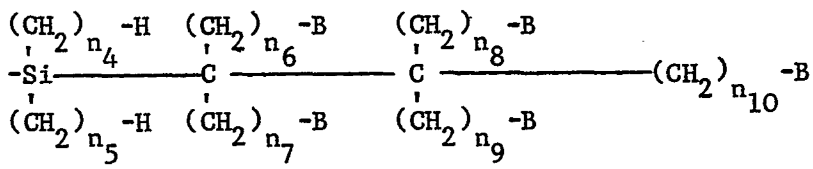 or

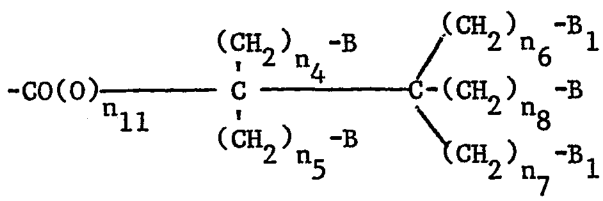

and wherein

$n_4$, $n_5$, $n_6$, $n_7$, $n_8$, $n_9$ and $n_{10}$ denote a number from 0 to 3,

$n_{11}$ denotes the number 0 or 1,

$B_1$ denotes B, $C_1$-$C_5$-alkoxy or $C_3$-$C_5$-alkenyl and

B, D and E denote hydrogen or phenyl which is optionally monosubstituted or disubstituted by nitro, halogen, $C_1$-$C_4$-alkyl, $C_1$-$C_4$-alkoxy or phenyl and

$R_7$ denotes phenyl diphenylmethyl or triphenylmethyl which is optionally monosubstituted to trisubstituted by $CF_3$, $NO_2$, $C_1$-$C_4$-alkyl, $C_1$-$C_4$-alkoxy, $C_1$-$C_4$-alkoxycarbonyl or phenyl or monosubstituted to pentasubstituted by halogen.

**3.** Process according to Claim 1 or 2, characterised in that compounds of the general formula

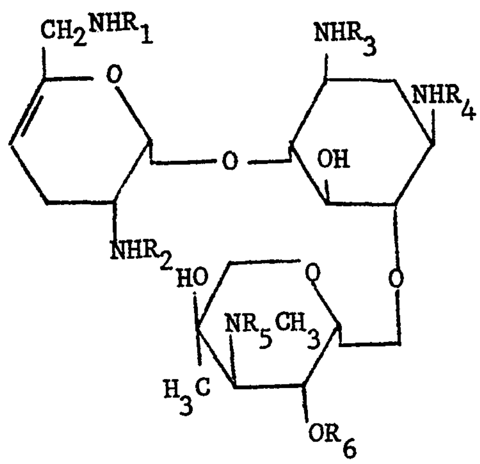

wherein

$R_1$, $R_2$, $R_3$, $R_4$, $R_5$ and $R_6$ have the meaning given in Claim 1 are used as starting compounds.

**4.** Use of the compounds prepared according to Claims 1 to 3 for the preparation of 4,6-di-0-(aminoglycosyl)-1,3-diamino-cyclitols.

**Revendications** (pour les Etats contractants : BE, CH, DE, FR, GB, IT, NL, SE)

**1.** Composés de formule générale

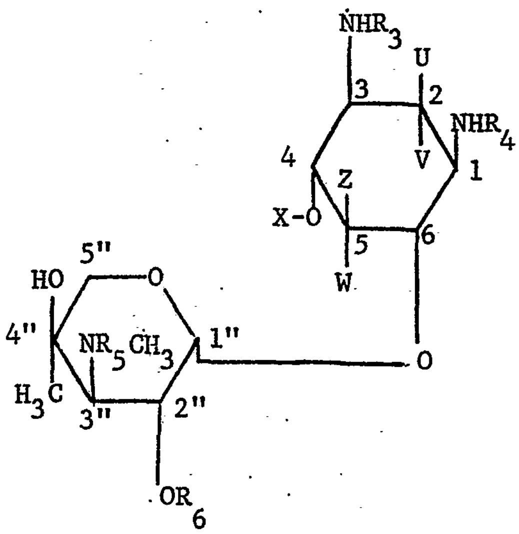

dans laquelle

X représente un reste de formule

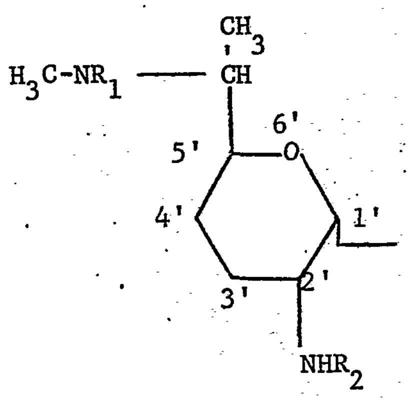

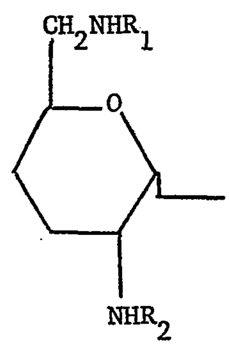

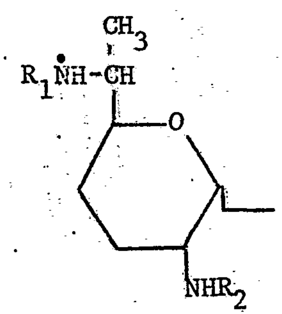

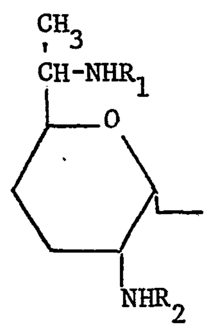

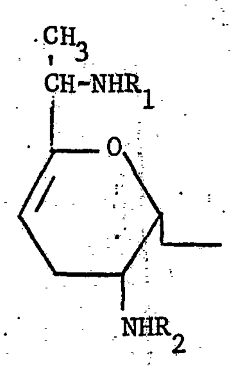

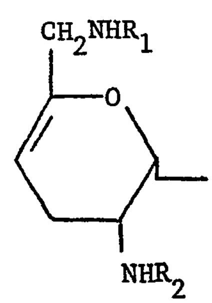

U, V, W et Z représentent l'hydrogène ou des groupes hydroxy,

$R_1$, $R_2$ et $R_3$ représentent $-CO-A$,

$R_4$ représente l'hydrogène,

$R_5$ représente un reste $-SR_7$ et

$R_6$ représente l'hydrogène ou un substituant encombrant éliminable, U et V d'une part, W et Z d'autre part, ne pouvant représenter simultanément OH,

A représente un reste de formule

$-CHal_3$, $-(CH_2)_nB$, $-O-E$, 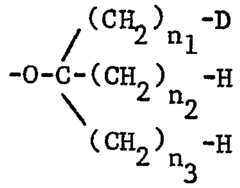 ou 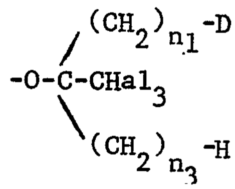

B et D représentent l'hydrogène ou un groupe phényle éventuellement substitué,

E représente un groupe phényle éventuellement substitué,

n est un nombre allant de 0 à 5,

$n_1$, $n_2$ et $n_3$ sont des nombres allant de 0 à 5,

Hal représente le fluor, le chlore ou le brome et

$R_7$ représente un groupe phényle, di- ou tri-phénylméthyle éventuellement substitué.

**2.** Composés selon la revendication 1, dans lesquels $R_6$ représente l'hydrogène ou un reste de formule

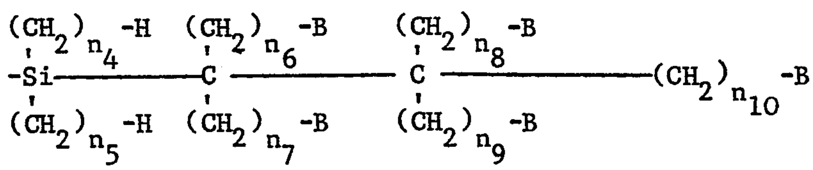 ou

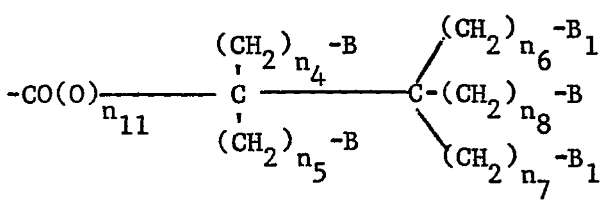

dans lesquelles

$n_4$, $n_5$, $n_6$, $n_7$, $n_8$, $n_9$, $n_{10}$ sont des nombres allant de 0 à 3,

$n_{11}$ est égal à 0 ou 1,

$B_1$ représente B, un groupe alcoxy en $C_1$-$C_5$ ou alcényloxy en $C_3$-$C_5$ et

B, D et E représentent l'hydrogène ou un groupe phényle éventuellement mono- ou di-substitué par des groupes nitro, des halogènes, des groupes alkyle en $C_1$-$C_4$, alcoxy en $C_1$-$C_4$ ou phényle et

$R_7$ représente un groupe phényle, diphénylméthyle ou triphénylméthyle éventuellement mono- à tri-substitué par des groupes $CF_3$, $NO_2$, alkyle en $C_1$-$C_4$, alcoxy en $C_1$-$C_4$, (alcoxy en $C_1$-$C_4$) carbonyle ou phényle ou mono- à penta-substitué par des halogènes.

**3.** Composés selon la revendication 1, de formule générale

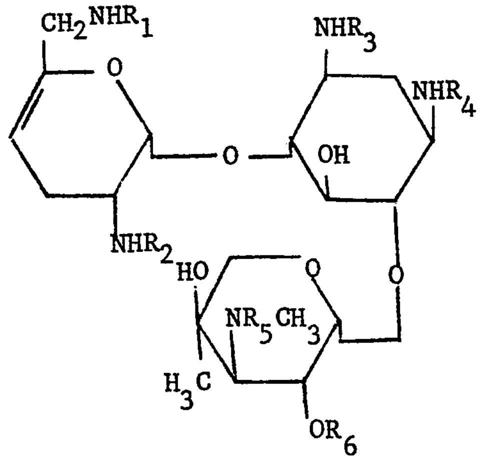

dans laquelle

$R_1$, $R_2$, $R_3$, $R_4$, $R_5$ et $R_6$ ont les significations indiquées dans la revendication 1.

**4.** Procédé de préparation des composés selon la revendication 1, caractérisé en ce que, partant de composés de formule

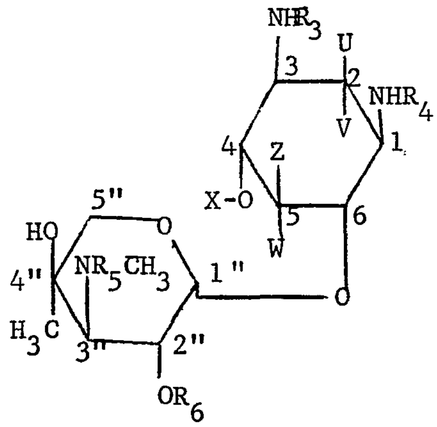

dans laquelle

$R_1$, $R_2$, $R_3$, $R_4$ et $R_5$ représentent un groupe $-SR_7$ et

$R_6$ représente l'hydrogène et

U, V, W, Z et $R_7$ ont les significations indiquées dans la revendication 1,

1. on fait réagir avec un agent silylant ou un agent acylant de formule

$$R_6-G$$

dans laquelle

$R_6$ représente un substituant encombrant éliminable et

G représente un groupe éliminable,

2. on élimine les groupes $-S-R_7$ représentés par $R_1$, $R_2$, $R_3$ et éventuellement $R_4$,

3. on acyle les groupes amino libérés à l'exception du groupe $1-NH_1$ lorsque celui-ci a déjà été libéré en 2) ci-dessus, à l'aide d'agents acylants de formule

$$A-CO-G \qquad (IV)$$

dans laquelle

A a les significations indiquées dans la revendication 1 et

G représente un groupe éliminable,

4. lorsque cela n'a pas déjà été fait sous 2), on élimine le groupe $S-R_7$ représenté par $R_4$ et, le cas échéant,

5. on élimine le substituant encombrant $R_6$, les opérations décrites sous 4) et 5) pouvant être réalisées dans l'ordre inverse.

**5.** Utilisation des composés selon la revendication 1 pour la préparation des 4,6-di-O-(aminoglycosyl)-1,3-diaminocyclitols.

**Revendications** (pour l'Etat contractant : AT)

**1.** Procédé pour la préparation de composés de formule générale

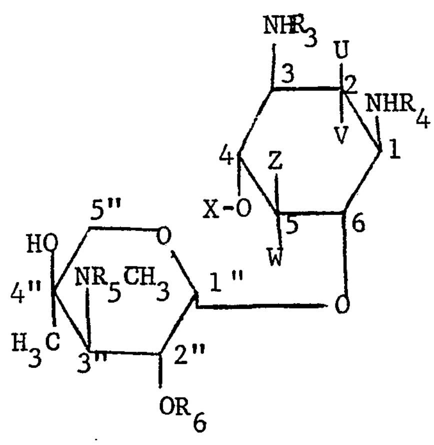

dans laquelle

X représente l'un des restes de formules

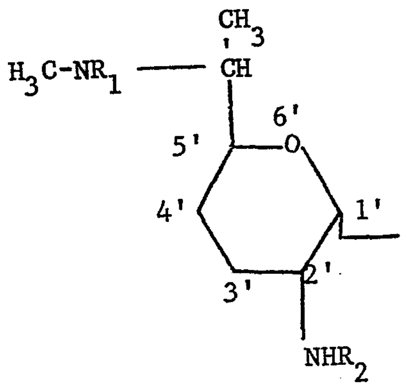

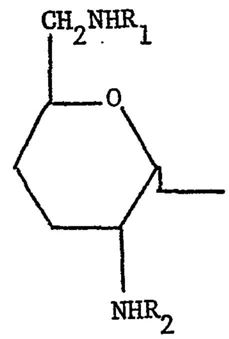

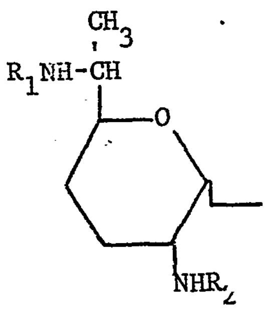

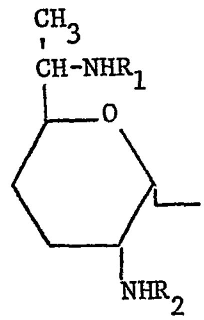

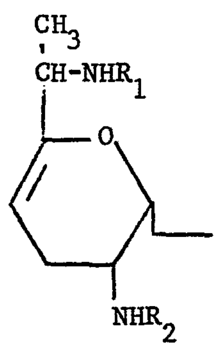

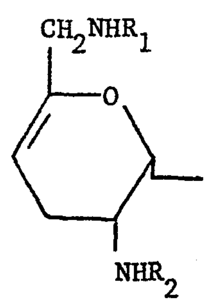

U, V, W et Z représentent l'hydrogène ou un reste hydroxy,

$R_1$, $R_2$ et $R_3$ représentent un groupe $-CO-A$,

$R_4$ représente l'hydrogène,

$R_5$ représente un reste $-SR_7$ et

$R_6$ représente l'hydrogène ou un substituant encombrant éliminable, U et V d'une part, W et Z d'autre part, ne pouvant représenter simultanément OH et

A représente un reste de formule

$-CHal_3$, $-(CH_2)_nB$, $-O-E$, 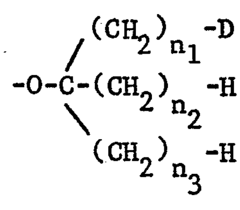 ou 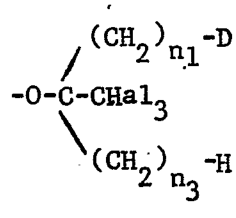

B et D représentent l'hydrogène ou un groupe phényle éventuellement substitué,

E représente un groupe phényle éventuellement substitué,

n est un nombre allant de 0 à 5,

$n_1$, $n_2$ et $n_3$ sont des nombres allant de 0 à 5,

Hal représente le fluor, le chlore ou le brome et

$R_7$ représente un groupe phényle, di- ou triphénylméthyle éventuellement substitué, caractérisé en ce que :

1. on fait réagir des composés de formule

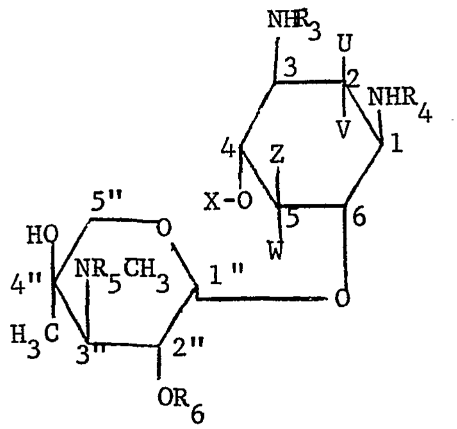

dans laquelle

$R_1$, $R_2$, $R_3$, $R_4$ et $R_5$ représentent un groupe $-SR_7$,

$R_6$ représente l'hydrogène et

X, U, V, W et Z sont tels que définis ci-dessus, avec un agent silylant ou un agent acylant de formule

$$R_6-G$$

dans laquelle

$R_6$ représente un substituant encombrant éliminable et

G représente un groupe éliminable,

2. on élimine les groupes $-S-R_7$, représentés par $R_1$, $R_2$, $R_3$ et éventuellement $R_4$,

3. on acyle les groupes amino libérés, à l'exception du groupe 1-$NH_2$, lorsque celui-ci a déjà été libéré dans l'étape 2, avec des agents acylants de formule

$$A-CO-G \qquad (IV)$$

dans laquelle

A est tel que défini ci-dessus et

G représente un groupe éliminable,

4. lorsque cela n'a pas déjà été fait sous 2, on élimine le groupe $S-R_7$ représenté par $R_4$ et, le cas échéant,

5. on élimine le substituant encombrant $R_6$, les étapes décrites sous 4 et 5 pouvant être effectuées dans l'ordre inverse.

**2.** Procédé selon la revendication 1, caractérisé en ce que $R_6$ représente l'hydrogène ou un reste de formule

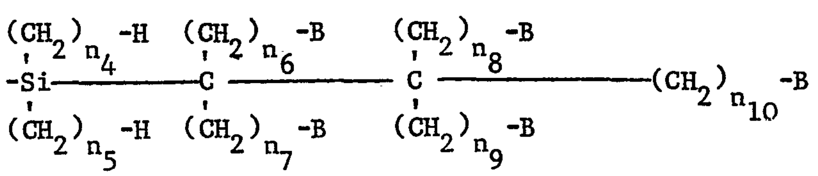 ou

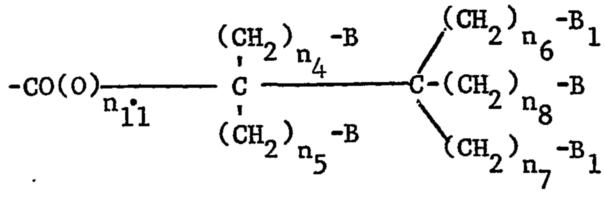 et

dans lesquelles

$n_4$, $n_5$, $n_6$, $n_7$, $n_8$, $n_9$, $n_{10}$ représentent des nombres de 0 à 3

$n_{11}$ est égal à 0 ou à 1,

$B_1$ représente B, un groupe alcoxy en $C_1$-$C_5$ ou alcényloxy en $C_3$-$C_5$ et

B, D et E représentent l'hydrogène ou un groupe phényle éventuellement mono- ou disubstitué par des groupes nitro, des halogènes, des groupes alkyle en $C_1$-$C_4$, alcoxy en $C_1$-$C_4$ ou phényle, et

$R_7$ représente un groupe phényle, diphénylméthyle ou triphénylméthyle éventuellement mono- à tri-substitué par des groupes $CF_3$, $NO_2$, alkyle en $C_1$-$C_4$, alcoxy en $C_1$-$C_4$ (alcoxy en $C_1$-$C_4$) carbonyle ou phényle, ou mono- à pentasubstitué par des halogènes.

**3.** Procédé selon la revendication 1 ou 2, caractérisé en ce que l'on part de composés de formule générale

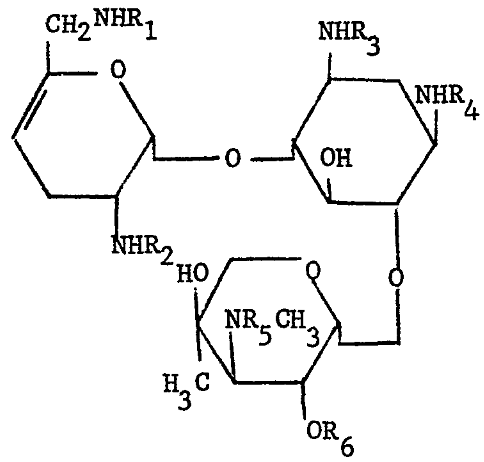

dans laquelle

$R_1$, $R_2$, $R_3$, $R_4$, $R_5$ et $R_6$ ont la signification indiquée à la revendication 1.

**4.** Utilisation des composés préparés selon les revendications 1 à 3 pour la préparation de 4,6-di-O-(aminoglycosyl)-1,3-diamino-cyclitols.